# EUROPEAN PATENT APPLICATION

(11) **EP 4 850 762 A1**
(43) Date of publication of application: **09.12.2026**
(21) Application number: 25181321.8
(22) Date of filing: 06.06.2025
(51) Int. Cl.: A61P 29/00, C07D 233/64, C07D 401/04, C07D 401/12, C07D 403/04, C07D 405/04, C07D 405/14, A61K 31/4178, A61K 31/4439, A61K 31/437

(54) **SUBSTITUTED IMIDAZOLES AS INHIBITORS OF NAV1.8**

(71) Applicant: Grünenthal GmbH, 52078 Aachen (DE)
(72) Inventor: NORDHOFF, Sonja, 52078 Aachen (DE); KRÜGER, Sebastian, 52078 Aachen (DE); MARIGO, Mauro, 52078 Aachen (DE); MÜLBAIER, Marcel, 52078 Aachen (DE)
(74) Representative: Grünenthal GmbH

(57) **Abstract**

The invention relates to compounds according to general formula (I) 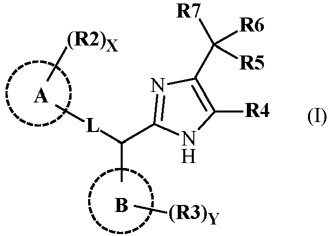 which act as inhibitors of Na_{V}1.8 and can be used in the treatment of pain.

## Description

The invention relates to compounds according to general formula (I) 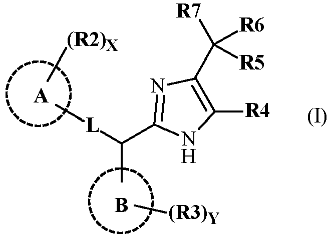 which act as inhibitors of Na_{V}1.8 and can be used in the treatment of pain.

Normal pain sensation (nociception) serves primarily as a survival mechanism, the body's way of self-protection, alerting against (further) tissue damage and disease from noxious stimuli. For instance, acute pain can arise when the external environment (temperature, pressure, chemicals) activates modality specific receptors (no-ciceptors) and ion channels within the skin. The peripheral terminals of pain-signaling neurons - whose cell bodies are found in the dorsal root ganglia [DRG] and trigeminal ganglia [TG] - convert the external stimuli into electrochemical generator potentials. Specific voltage-gated sodium channels (Na_{V}s) integrate and amplify these generator signals until the threshold for an action potential [AP] is reached. Thus, what starts as a noxious stimuli in the periphery eventually leads to action potential firing that travels towards the central nervous system, synapsing first onto neurons in the spinal cord and then towards the brain. Na_{V}s also function to support propagation of action potentials to the central terminals within the spinal cord. At the end of its travels along the somatosensory pathway, the action potential signal is interpreted as pain by the brain (Lumpkin and Caterina, Nature (2007), Vol.445 pp 858-865; and Crawford and Caterina Toxicologic Pathology (2020) 48(1)-174; Goodwin G and McMahon S.B. Nature Reviews Neuroscience (2021) Vol 22 pp 263-274; Bennett D.L. et al. Physiol Rev 99 (2019) Vol 99 pp 1079-1151).

Abnormal persistent neuropathic pain arises as a consequence of a lesion or disease of this somatosensory pathway. In response to nerve injury or inflammation, abnormal changes in ion channel expression can cause hyper-excitability of pain-signaling neurons and their nerves/axons, thus resulting in pathological pain.

The voltage-gated Na_{V}1.8 sodium channel is a therapeutic target for analgesia because of its restricted expression profile (almost exclusive to peripheral sensory tissues), its placement along the pain pathway (free nerve endings, sciatic nerve, and DRG), prominent physiological role in pain signaling (supports upstroke of AP and facilitates repetitive AP firing), and supporting genetic/pharmaco-phenotypic evidence (human/animal studies showing changes in Na_{V}1.8 function cause parallel changes in pain sensitivity).

Regarding its expression profile along the pain pathway, because Na_{V}1.8 was first found predominantly in peripheral sensory neurons of the dorsal root ganglia (DRG) and trigeminal ganglia (TG), it was originally termed SNS (sensory neuron specific) (Akopian A.N. et al Nature (1996) Vol 379 pp 257-261) or PN3 (peripheral nerve 3) (Sangameswaran L. et al J. Biol. Chem. (1996) Vol 271 pp 5953-5956). As well, Na_{V}1.8 is localized at free nerve endings, where pain signaling is initiated in the skin (Persson A.K. et al Mol. Pain. (2010) 6:84) and is diffusely localized along the entire length of non-myelinated axons of sciatic nerve (Rush A.M. et al. Eur.J.Neurosci (2005) Vol 22 pp 39-49).

In contrast, Na_{V}1.8 has minimal expression in nonneuronal tissue, such as heart and skeletal muscle, and in the CNS, including brain and spinal cord (9, 10, 338, 406) (Akopian A.N. op. cit.; Akopian A.N. et al. Nat. Neurosci (1999) Vol 2 pp 541-548; Novakovic S.D. et al. J. Neurosci. (1998) Vol 18, pp 2174-2187; and Sagameswaran L. op. cit.).

Regarding its physiological role, Na_{V}1.8 contributes the majority of the inward current during the rising phase of an all-or-none action potential in nociceptive sensory neurons (Blair N.T. et al. J. Neurosci. (2003) Vol 23 pp 10338-10350 and Renganathan M et al. J. Neurophysiol (2001) Vol 86 pp 629-640) - and also contributes most of the current in subsequent spikes during repetitive firing in DRG neurons (Choi J.S. J. Neurophysiol (2011) Vol 106 pp 3173-3184; and Tan Z.Y. et al. J. Neurosci. (2014) Vol 34 pp 7190-7197).

Regarding genetic and pharmacology studies, gain-of-function mutations in Na_{V}1.8 were found in patients with chronic neuropathic pain such as small fiber neuropathy (Faber C.G. et al. (2012) Ann. Neurol Vol 71 pp 26-39; Han C et al. J. Neurol Neurosurg Psychiatry (2014) Vol 85 pp 499-505; and, Kist A.M. et al. PLoS One (2016) Vol 11 e0161789); Eijkenboom I. et al J. Neurol Neurosurg Psychiatry (2019) 90 (3) pp 342-352); loss-of-function (gene knockout) studies in mice reduced pain sensitivity, notably in nociception (Laird J.M. et al. J. Neurosci

(2002) J. Neurosci Vol 22 pp 8352-8356; Jarvis M.F. et al Proc Natl Acad Sci USA (2007) Vol 104 pp 8520-8525; Joshi S.K. et al Pain (2006) Vol 123 pp 75-82) and in neuropathic models (Roza C. et al J Physiol (2003) Vol 550 pp 921-926); Na_{V}1.8-selective small molecule inhibitors reduced pain in rodents, specifically in inflammatory and neuropathic models (Jarvis et al. op. cit.; Kort M.E. et al Bioorg Med Chem Lett (2010) Vol 20 pp 6812-6815; Scanio M.J. et al Bioorg Med Chem (2010) Vol 18 pp 7816-7825; Payne C.E. et al Br J Pharmacol (2015) Vol 172 pp 2654-2670).

Currently, non-selective Na_{V} channel inhibitors are used to treat epilepsy, cardiac arrhythmia, and chronic pain (Hille, B. J. Gen. Physiol. (1977) Vol. 69 pp. 497-515; Hille. B, Ion Channels of Excitable Membranes (1992) pp. 391-421; Sunderland, Mass., Sinauer Associates, Inc. 3rd ed.; Hondeghem L.M. and Katzung B.G. Annu. Rev. Pharmacol. Toxicol. (1984) Vol. 24. Pp. 387-423; Catterall W.A. Trends Pharmacol. Sci. (1987) Vol. 8 pp.57-65) - however, all of these analgesics have limited efficacy owing to dose-limiting adverse side-effects related to inhibiting Na_{V}1.1/Na_{V}1.2/1.6 (seizure liability), inhibiting Na_{V}1.4 (muscle weakness/paralysis), inhibiting Na_{V}1.5 (arrhythmia risk).

There is a need to develop a Na_{V}1.8-selective small molecule inhibitor as an effective and safe analgesic.

It was an object of the invention to provide novel compounds which are inhibitors, preferably selective inhibitors, of Na_{V}1.8, and which preferably have advantages over the compounds of the prior art. The novel compounds should in particular be suitable for use in the treatment of pain.

This object has been achieved by the subject-matter of the patent claims.

It was surprisingly found that the compounds according to the invention are highly potent and selective inhibitors of the Na_{V}1.8 channel. Further it was surprisingly found that the compounds according to the invention have advantageous properties compared to the Na_{V}1.8 inhibitors of the prior art.

A first aspect of the invention relates to a compound according to general formula (I) 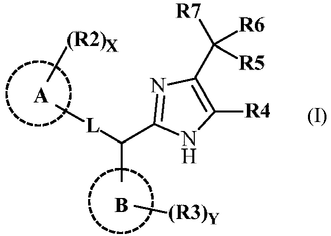 wherein
- **A** and **B**: independently from one another represent phenyl, 5 to 10-membered heteroaryl, C₃₋₇-cycloalkyl, or 4- to 10-membered heterocycloalkyl;
- **R2** and **R3**: independently from one another represent F, Cl, CN, C₁₋₄-alkyl, C₃₋₇-cycloalkyl, OH, OC₁₋₄-alkyl, NH₂, NHC₁₋₄-alkyl or N(C₁₋₄-alkyl)₂;
- **X** and **Y**: independently from one another represent 0, 1, 2, 3 or 4;
- **R4**: represents H, Cl, CN, C₁₋₄-alkyl or C₃₋₇-cycloalkyl;
- **L**: represents bond, O, NH or C₁₋₃-alkylene;
- **R5**: represents H or C₁₋₄-alkyl;
- **R6**: represents H, C₁₋₄-alkyl, 4- to 7-membered heterocycloalkyl, or C₁₋₃-alkylene-(4- to 7-membered heterocycloalkyl); or
- **R5** and **R6**: together with the carbon atom to which they are attached form C₃₋₇-cycloalkyl or 4- to 7-membered heterocycloalkyl; and
- **R7**: represents OH, OC₁₋₄-alkyl, O-4- to 7-membered heterocycloalkyl, NH₂, NHC₁₋₄-alkyl, N(C₁₋₄-alkyl)₂, or N**RaRb** wherein **Ra** and **Rb** together with the nitrogen atom to which they are attached form 4- to 7-membered heterocycloalkyl;

wherein C₁₋₄-alkyl and C₁₋₃-alkylene in each case independently from one another is linear or branched, saturated or unsaturated;
wherein C₁₋₄-alkyl, C₁₋₃-alkylene, C₃₋₇-cycloalkyl, 4- to 7-membered heterocycloalkyl and 4- to 10-membered heterocycloalkyl in each case independently from one another are unsubstituted or mono- or polysubstituted with one or more substituents selected from F, C₁₋₄-alkyl, C₁₋₃-alkylene-NH₂, CH₂F, CHF₂, CF₃, OH, OC₁₋₄-alkyl, OCH₂F, OCHF₂, OCF₃, NH₂, NHC₁₋₄-alkyl and N(C₁₋₄-alkyl)₂;
wherein phenyl and 5 to 10-membered heteroaryl in each case independently from one another are unsubstituted or mono- or polysubstituted with one or more substituents selected from F, Cl, CN, C₁₋₄-alkyl, CH₂F, CHF₂, CF₃, OH, OCH₂F, OCHF₂, OCF₃, OC₁₋₄-alkyl, NH₂, NHC₁₋₄-alkyl and N(C₁₋₄-alkyl)₂;
in the form of the free compound or a physiologically acceptable salt thereof.

In a preferred embodiment, the compound according to the invention is present in form of the free compound. For the purpose of specification, "free compound" preferably means that the compound according to the invention is not present in form of a salt. Methods to determine whether a chemical substance is present as the free compound or as a salt are known to the skilled artisan such as ¹⁴N or ¹⁵N solid state NMR, x-ray diffraction, x-ray powder diffraction, IR, Raman, XPS. ¹H-NMR recorded in solution may also be used to consider the presence of protonation.

In another preferred embodiment, the compound according to the invention is present in form of a physiologically acceptable salt. For the purposes of this specification, the term "physiologically acceptable salt" preferably refers to a salt obtained from a compound according to the invention and a physiologically acceptable acid or base.

According to the invention, the compound according to the invention may be present in any possible form including solvates, cocrystals and polymorphs. For the purposes of this specification, the term "solvate" preferably refers to an adduct of (i) a compound according to the invention and/or a physiologically acceptable salt thereof with (ii) distinct molecular equivalents of one or more solvents.

Further, the compound according to the invention may be present in form of the racemate, enantiomers, diastereomers, tautomers or any mixtures thereof. The person skilled in the art knows that imidazoles show annular tautomerism.

The compounds according to the invention may have one or more stereocenter. The person skilled in art knows by looking at a chemical structure whether the depicted compound has one or more stereocenters or not.

For some compounds according to the invention that have one or more stereocenters and which chemical structures are disclosed in the examples of the present application, the chemical structure includes bold bonds and/or hashed bonds to indicate the relative structural orientation of those substituents connected by the bold bonds and/or hashed bonds to the superior structure. If the bold bonds and/or hashed bonds are depicted in form of a wedge, the absolute stereochemical configuration of the compound is known and thereby indicated. If the bold bonds and/or hashed bonds are depicted as a straight bond (i.e. no wedge), the absolute stereochemical configuration of the compound has not been determined. In that case, the bold bonds and/or hashed bonds merely serve to indicate that this particular compound is present as one enantiomer or one diastereomer (e.g. cis-diastereomer (i.e. mixture of two cis-enantiomers) or trans-diastereomer (i.e. mixture of two trans-enantiomers)). All compounds according to the invention that have one or more stereocenters but which chemical structures disclosed in the examples of the present application do not include bold bonds and/or hashed bonds, are present as a mixture of the respective stereoisomers.

The invention also includes isotopic isomers of a compound of the invention, wherein at least one atom of the compound is replaced by an isotope of the respective atom which is different from the naturally predominantly occurring isotope, as well as any mixtures of isotopic isomers of such a compound. Preferred isotopes are ²H (deuterium), ³H (tritium), ¹³C and ¹⁴C. Isotopic isomers of a compound of the invention can generally be prepared by conventional procedures known to a person skilled in the art.

According to the invention, the term "C₁₋₄-alkyl" preferably means acyclic and preferably saturated hydrocarbon residues, which can be linear (i.e. unbranched) or branched and which can be unsubstituted or mono- or polysubstituted (e.g. di- or trisubstituted), and which contain 1 to 4 (i.e. 1, 2, 3 or 4) carbon atoms. Preferably, C₁₋₄-alkyl is saturated. Preferred C₁₋₄-alkyl groups are selected from the group consisting of methyl, ethyl, n-propyl, 2-propyl, n-butyl, isobutyl, sec-butyl and tert-butyl.

Still further according to the invention, the term "C₃₋₇-cycloalkyl" preferably means monocyclic or polycyclic, preferably monocyclic or bicyclic, aliphatic hydrocarbons containing 3 to 7 (i.e. 3, 4, 5, 6 or 7) carbon atoms, wherein the hydrocarbons in each case can be saturated or unsaturated (but not aromatic), unsubstituted or mono- or polysubstituted.

For the purpose of this specification, "polycyclic" and "bicyclic" shall preferably mean fused, bridged or spiro cyclic systems.

Preferably, C₃₋₇-cycloalkyl is saturated. C₃₋₇-cycloalkyl can be bound to the respective superordinate general structure via any desired and possible ring member of the cycloalkyl group. C₃₋₇-cycloalkyl can also be fused with further saturated or (partially) unsaturated heterocycloalkyl, aromatic or heteroaromatic ring systems, preferably aromatic ring system, which can in turn be unsubstituted or mono- or polysubstituted, thereby being part of a bi- or polycyclic system having up to 14 ring members. Accordingly, the term "C₃₋₇-cycloalkyl" preferably includes monocyclic or polycyclic, preferably monocyclic or bicyclic, aliphatic hydrocarbons containing 3 to 7 carbon atoms; which are fused with a phenyl moiety. Preferred C₃₋₇-cycloalkyl groups are selected from the group consisting of cyclopropyl, cyclobutyl, cyclopentyl, cyclohexyl, cycloheptyl, spiro[2.2]pentyl, spiro[2.3]hexyl, spiro[3.3]heptyl, bicyclo[1.1.0]butyl, bicyclo[2.1.0]pentyl, bicyclo[2.1.1]hexyl, bicyclo[3.1.1]heptyl, bicyclo[2.2.1]heptyl, bicyclo[3.1.0]hexyl, bicyclo[3.2.0]heptyl, and bicyclo[4.1.0]heptyl.

Further according to the present invention, the term "C₁₋₃-alkylene" relates to linear or branched, preferably linear, and preferably saturated aliphatic residues which are preferably selected from the group consisting of methylene (-CH₂-), ethylene (-CH₂CH₂-), propylene (-CH₂CH₂CH₂- or -C(CH₃)₂-), more preferably methylene (-CH₂-) and ethylene (-CH₂CH₂-).

According to the invention, C₁₋₄-alkyl and C₁₋₃-alkylene in each case independently from one another is linear or branched, saturated or unsaturated. In a preferred embodiment, C₁₋₄-alkyl and C₁₋₃-alkylene in each case independently from one another is linear or branched, and saturated.

According to the present invention, the terms "4 to 7-membered heterocycloalkyl" and "4 to 10-membered heterocycloalkyl" preferably mean monocyclic or polycyclic, preferably monocyclic or bicyclic, heterocycloaliphatic saturated or unsaturated (but not aromatic) residues having 4 to 7 (i.e. 4, 5, 6 or 7) or 4 to 10 (i.e. 4, 5, 6, 7, 8, 9 or 10) ring members wherein in each case at least one, if appropriate also two or three carbon atoms are replaced by a heteroatom or a heteroatom group each selected independently of one another from the group consisting of O, S, S(=O), S(=O)₂, N, NH and N(C₁₋₄-alkyl) such as N(CH₃), wherein the carbon atoms of the ring can be unsubstituted or mono- or polysubstituted. Preferably, 4 to 7-membered heterocycloalkyl and 4 to 10-membered heterocycloalkyl are saturated. The 4 to 7-membered heterocycloalkyl and the 4 to 10-membered heterocycloalkyl groups can also be fused with further saturated or (partially) unsaturated cycloalkyl, aromatic or heteroaromatic ring systems, preferably aromatic ring system, which can in turn be unsubstituted or mono- or polysubstituted, if not indicated otherwise, thereby being part of a bi- or polycyclic system having up to 14 ring members.. In a preferred embodiment, 4 to 7-membered heterocycloalkyl and 4 to 10-membered heterocycloalkyl are not fused with further ring systems. Still more preferably, 4 to 7-membered heterocycloalkyl and 4 to 10-membered heterocycloalkyl are not fused with further ring systems and are saturated. In another preferred embodiment, 4 to 7-membered heterocycloalkyl and 4 to 10-membered heterocycloalkyl are condensed with further ring systems, preferably phenyl. The 4 to 7-membered heterocycloalkyl and 4 to 10-membered heterocycloalkyl group can be bound to the superordinate general structure via any desired and possible ring member of the heterocycloaliphatic residue if not indicated otherwise. In a preferred embodiment, 4 to 7-membered heterocycloalkyl and 4 to 10-membered heterocycloalkyl are bound to the superordinate general structure via a carbon atom. In another preferred embodiment, 4 to 7-membered heterocycloalkyl and 4 to 10-membered heterocycloalkyl are bound to the superordinate general structure via a heteroatom, in particular N.

Preferred 4 to 7-membered heterocycloalkyl groups are selected from the group consisting of 1,1-dioxo tetrahydrothiophenyl, 1-oxo thiomorpholinyl, tetrahydropyranyl, oxetanyl, tetrahydrofuranyl, morpholinyl, pyrrolidinyl, pyrrolidinonyl, azetidinyl, piperazinyl, piperazinonyl, piperidinyl, thietanyl, 1,1-dioxothietanyl, 2,6-di-azaspiro[3.3]heptyl, 2,5-diazabicyclo[2.2.1]heptyl, 3,6-diazabicyclo[3.3.1]heptyl, azepanyl, dioxepanyl, oxazepanyl, diazepanyl, thiazolidinyl, tetrahydrothiophenyl, tetrahydropyridinyl, thiomorpholinyl, 4-methylpiperazinyl, morpholinonyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, pyrazolidinyl, and cromanyl.

Preferred 4 to 10-membered heterocycloalkyl groups are selected from the group consisting of tetrahydro-2H-thiopyranyl 1,1-dioxide, 1-oxo thiomorpholinyl, tetrahydropyranyl, oxetanyl, tetrahydrofuranyl, morpholinyl, pyrrolidinyl, pyrrolidinonyl, azetidinyl, piperazinyl, piperazinonyl, piperidinyl, thietanyl, 1,1-dioxothietanyl, 2,6-diazaspiro[3.3]heptyl, 2,5-diazabicyclo[2.2.1]heptyl, 3,6-diazabicyclo[3.3.1]heptyl, cromanyl, thiepanyl 1,1-dioxide, 1,2-thiazepanyl 1,1-dioxide, 2,5,6,7-tetrahydro-1,2-thiazepinyl 1,1-dioxide, 5,6-dihydro-2H-1,2-thiazinyl 1,1-dioxide, 1,1-dioxo tetrahydrothiophenyl, 2-oxaspiro[3.4]octyl, 5-oxaspiro[2.4]heptyl, 2-oxabicyclo[2.1.1]hexyl, 1,1-dioxo thietanyl, 2,3-dihydro-[1,4]dioxino[2,3-b]pyridyl, tetrahydro-1,8-naphthyridinyl, azepanyl, dioxepanyl, oxazepanyl, diazepanyl, thiazolidinyl, tetrahydrothiophenyl, tetrahydropyridinyl, thiomorpholinyl, 4-methylpiperazinyl, morpholinonyl, dithiolanyl, dihydropyrrolyl, dioxanyl, dioxolanyl, dihydropyridinyl, dihydrofuranyl, dihydroisoxazolyl, dihydrooxazolyl, imidazolidinyl, isoxazolidinyl, oxazolidinyl, pyrazolidinyl, 5,7-dihydrofuro[3,4-b]pyridinyl, and 5,7-dihydrofuro[3,4-d]pyrimidinyl; more preferably tetrahydro-2H-thiopyranyl 1,1-dioxide, 1-oxo thiomorpholinyl, tetrahydropyranyl, oxetanyl, tetrahydrofuranyl, pyrrolidinyl, pyrrolidinonyl, azetidinyl, piperazinyl, piperazinonyl, piperidinyl, 1,1-dioxothietanyl, 2,6-diazaspiro[3.3]heptyl, 2,5-diazabicyclo[2.2.1]heptyl, 3,6-diazabicyclo[3.3.1]heptyl, cromanyl, thiepanyl 1,1-dioxide, 1,2-thiazepanyl 1,1-dioxide, 2,5,6,7-tetrahydro-1,2-thiazepinyl 1,1-dioxide and 5,6-dihydro-2H-1,2-thiazinyl 1,1-dioxide.

According to the present invention, the term "5 to 10-membered heteroaryl" preferably means a 5, 6, 7, 8, 9 or 10-membered monocyclic or bicyclic aromatic residue, respectively, which contains at least 1, if appropriate also 2, 3, 4 or 5 heteroatoms, wherein the heteroatoms are each selected independently of one another from the group S, N and O and the heteroaryl residue can be unsubstituted or mono- or polysubstituted, if not indicated otherwise. In the case of substitution on the heteroaryl, the substituents can be the same or different and be in any desired and possible position of the heteroaryl. The binding to the superordinate general structure can be carried out via any desired and possible ring member of the heteroaryl residue if not indicated otherwise. Preferably, the 5 to 10-membered heteroaryl is bound to the supraordinate general structure via a carbon atom of the heterocycle. The heteroaryl can also be fused with further saturated or (partially) unsaturated cycloalkyl, or heterocycloalkyl ring systems, which can in turn be unsubstituted or mono- or polysubstituted, thereby being part of a bi- or polycyclic system having up to 14 ring members. In a preferred embodiment, the 5 to 10-membered heteroaryl is not fused with further saturated or (partially) unsaturated cycloalkyl, or heterocycloalkyl ring systems. Preferably, the 5 to 10-membered heteroaryl is selected from the group consisting of pyridyl (i.e. 2-pyridyl, 3-pyridyl, 4-pyridyl), pyrimidinyl, pyridazinyl, pyrazinyl, pyrrolyl, imidazolyl, pyrazolyl, oxazolyl, isoxazolyl, thiazolyl, isothiazolyl, furanyl, thienyl (thiophenyl), triazolyl, thiadiazolyl, 4,5,6,7-tetrahydro-2H-indazolyl, 2,4,5,6-tetrahydrocyclopenta[c]pyrazolyl, benzofuranyl, benzoimidazolyl, benzothienyl, benzothiadiazolyl, benzothiazolyl, benzotriazolyl, benzooxazolyl, benzooxadiazolyl, quinazolinyl, quinoxalinyl, carbazolyl, quinolinyl, dibenzofuranyl, diben-zothienyl, imidazothiazolyl, indazolyl, indolizinyl, indolyl, isoquinolinyl, naphthyridinyl, oxazolyl, oxadiazolyl, phenazinyl, phenothiazinyl, phthalazinyl, purinyl, phenazinyl, tetrazolyl and triazinyl. Particularly preferred is pyridyl (i.e. 2-pyridyl, 3-pyridyl, 4-pyridyl).

In connection with the terms "C₁₋₄-alkyl", "C₁₋₃-alkylene", "C₃₋₇-cycloalkyl", "4- to 7- membered heterocycloalkyl" and "4- to 10- membered heterocycloalkyl" the term "substituted" refers in the sense of the invention, with respect to the corresponding residues or groups, to the single substitution (monosubstitution) or multiple substitution (poly substitution), e.g. disubstitution, trisubstitution or tetrasubstitution; more preferably to monosubstitution, disubstitution or trisubstitution; of one or more hydrogen atoms each independently of one another by at least one substituent. In case of a multiple substitution, i.e. in case of polysubstituted residues, such as di- or trisubstituted residues, these residues may be polysubstituted either on different or on the same atoms, for example trisubstituted on the same carbon atom, as in the case of CF₃, CH₂CF₃ or disubstituted as in the case of 1,1-difluo-rocyclopropyl, or at various points, as in the case of 1-chloro-3-fluorocyclopropyl. The multiple substitution can be carried out using the same or using different substituents.

If a residue occurs multiply within a molecule, then this residue can have respectively different meanings for various substituents.

According to the invention, C₁₋₄-alkyl, C₁₋₃-alkylene, C₃₋₇-cycloalkyl, 4- to 7- membered heterocycloalkyl and 4- to 10- membered heterocycloalkyl in each case independently from one another are unsubstituted or mono- or polysubstituted with one or more substituents selected from F, C₁₋₄-alkyl, C₁₋₃-alkylene-NH₂, CH₂F, CHF₂, CF₃, OH, OC₁₋₄-alkyl, OCH₂F, OCHF₂, OCF₃, NH₂, NHC₁₋₄-alkyl and N(C₁₋₄-alkyl)₂.

Further according to the invention, phenyl and 5 to 10-membered heteroaryl in each case independently from one another are unsubstituted or mono- or polysubstituted with one or more substituents selected from F, Cl, CN, C₁₋₄-alkyl, CH₂F, CHF₂, CF₃, OH, OCH₂F, OCHF₂, OCF₃, OC₁₋₄-alkyl, NH₂, NHC₁₋₄-alkyl and N(C₁₋₄-alkyl)₂.

In a preferred embodiment, **R2** represents F, Cl, CH₃, CH₂F, CHF₂, CF₃ or OCH₃.

In a preferred embodiment, **R3** represents F, Cl, CH₃, CH₂F, CHF₂, CF₃ or OCH₃.

In a preferred embodiment, **R4** represents H, Cl, CN, CH₃, CH₂F, CHF₂, CF₃ or CH₂OCH₃.

In a preferred embodiment, **R5** represents H, CH₃ or CH₂OCH₃.

In a preferred embodiment, **R6** represents H, CH₃, CH₂F, CHF₂, CF₃, CH₂OH, CH₂OCH₃, CH₂OCH₂F, CH₂OCHF₂, CH₂OCF₃, CH₂NH₂, azetidine unsubstituted or CH₂-azetidine unsubstituted.

In a preferred embodiment, **R5** and **R6** together with the carbon atom to which they are attached form cycloalkyl selected from cyclobutyl; in each case unsubstituted or monosubstituted with NH₂; or heterocycloalkyl selected from oxetane, azetidine, oxolane, pyrrolidine, tetrahydropyran, and piperidine; in each case unsubstituted or monosubstituted with CH₃.

In a preferred embodiment, **R7** represents OH, OCH₃, O-azetidine unsubstituted, NH₂, NHCH₃, N(CH₃)₂, or N**RaRb** wherein **Ra** and **Rb** together with the nitrogen atom to which they are attached form azetidine unsubstituted.

In a preferred embodiment, **R7** represents OH, OC₁₋₄-alkyl or O-4- to 7-membered heterocycloalkyl.

In a preferred embodiment, **R7** represents NH₂, NHC₁₋₄-alkyl, N(C₁₋₄-alkyl)₂ or N**RaRb** wherein **Ra** and **Rb** together with the nitrogen atom to which they are attached form 4- to 7-membered heterocycloalkyl.

In preferred embodiments, **R5, R6** and **R7** together with the carbon atom to which they are attached represent -CH(NH₂)CH₃, -CH(NH₂)CHF₂, -CH(NH₂)CH₂OH, -CH(NHCH₃)CH₂OH, -CH(N(CH₃)₂)CH₂OH, -CH(NH₂)CH₂OCH₃, -CH(NH₂)CH₂OCHF₂, -CH(NH₂)CH₂OCF₃, -CH(NHCH₃)CH₂OCH₃, -CH(NHCH₃)CH₂OCHF₂, -CH(N(CH₃)₂)CH₂OCH₃, -C(NH₂)(CH₂OCH₃)₂, or -C(NH₂)(CH₃)CH₂OH.

In preferred embodiments, **R5, R6** and **R7** together with the carbon atom to which they are attached represent a residue selected from:

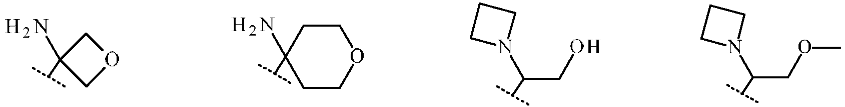

In preferred embodiments, **R5, R6** and **R7** together with the carbon atom to which they are attached represent -CH(OH)CH₃, -CH(OH)CH₂NH₂, -CH(OH)CH₂OH, -CH(OH)CH₂OCH₃, -CH(OCH₃)CH₂OH, -C(OH)(CH₂OCH₃)₂, -C(OH)(CH₃)₂, or -C(OH)(CH₃)CH₂OH.

In preferred embodiments, **R5, R6** and **R7** together with the carbon atom to which they are attached represent a residue selected from:

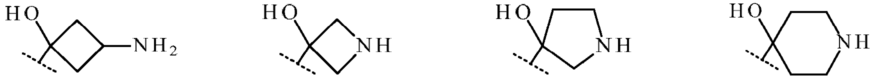

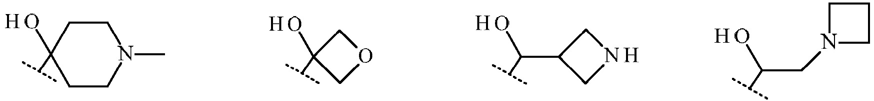

In a preferred embodiment, **L** represents bond, O or N(H).

In a preferred embodiment, **A** represents phenyl, pyridyl or cyclohexyl.

In a preferred embodiment, **B** represents phenyl.

In preferred embodiments, the compound according to the invention is according to general formula (II) or (III) 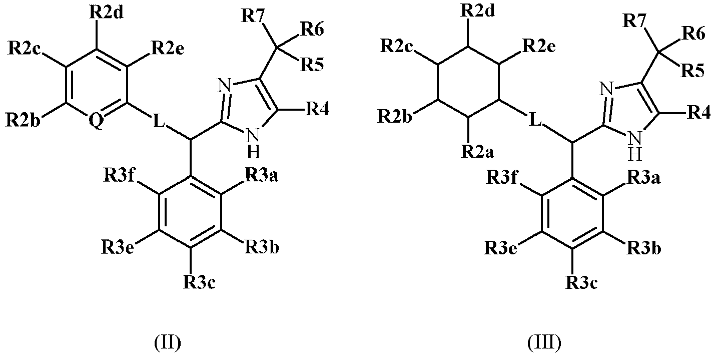 wherein
**L, R4, R5, R6** and **R7** are defined above;
**Q** represents N or C**R2a;**
**R2a, R2b, R2c, R2d** and **R2e** independently from one another represent H, F, Cl, CH₃, CH₂F, CHF₂, CF₃ or OCH₃; with the proviso that not more than 4 of **R2a, R2b, R2c, R2d,** and **R2e** are other than H;
**R3a, R3b, R3c, R3d** and **R3e** independently from one another represent H, F, Cl, CH₃, CH₂F, CHF₂, CF₃ or OCH₃; with the proviso that not more than 4 of **R3a, R3b, R3c, R3d** and **R3e** are other than H.

In preferred embodiments, the compound according to the invention is selected from the group consisting of
1 1-(2-(bis(3-chloro-4-fluorophenyl)methyl)-5-methyl-1H-imidazol-4-yl)ethane-1,2-diol
2 3-(2-(bis(3-chloro-4-fluorophenyl)methyl)-5-methyl-1H-imidazol-4-yl)azetidin-3-ol
3 1-(2-(bis(3-chloro-4-fluorophenyl)methyl)-4-methyl-1H-imidazol-5-yl)ethan-1-ol
4 1-(2-((3,4-difluorophenyl)((5-fluoro-6-methylpyridin-2-yl)amino)methyl)-5-methyl-1H-imidazol-4-yl)ethane-1,2-diol
5 1-(2-((3-chloro-4-fluorophenyl)((4,4-difluorocyclohexyl)oxy)methyl)-5-methyl-1H-imidazol-4-yl)ethane-1,2-diol
6 1-(2-((3-chloro-4-fluorophenyl)((5-fluoro-6-methylpyridin-2-yl)amino)methyl)-5-(methoxymethyl)-1H-imidazol-4-yl)ethane-1,2-diol
7 3-(2-(bis(3-chloro-4-fluorophenyl)methyl)-5-methyl-1H-imidazol-4-yl)pyrrolidin-3-ol
8 1-(2-((3-chloro-4-fluorophenyl)((5-fluoro-6-methylpyridin-2-yl)amino)methyl)-5-methyl-1H-imidazol-4-yl)ethane-1,2-diol
9 4-(2-(bis(3-chloro-4-fluorophenyl)methyl)-5-methyl-1H-imidazol-4-yl)piperidin-4-ol
10 2-(2-(bis(3-chloro-4-fluorophenyl)methyl)-4-methyl-1H-imidazol-5-yl)-1,3-dimethoxypropan-2-ol
11 2-amino-1-(2-((3-chloro-4-fluorophenyl)((5-fluoro-6-methylpyridin-2-yl)amino)methyl)-5-methyl-1H-imidazol-4-yl)ethan-1-ol
12 2-(2-(bis(3-chloro-4-fluorophenyl)methyl)-5-methyl-1H-imidazol-4-yl)propane-1,2-diol
13 2-(2-(bis(3-chloro-4-fluorophenyl)methyl)-5-methyl-1H-imidazol-4-yl)propan-2-ol
14 1-(2-(bis(4-chloro-2-methoxyphenyl)methyl)-5-methyl-1H-imidazol-4-yl)ethane-1,2-diol
15 4-(2-(bis(4-chloro-2-methoxyphenyl)methyl)-4-methyl-1H-imidazol-5-yl)piperidin-4-ol
16 2-amino-1-(2-(bis(3-chloro-4-fluorophenyl)methyl)-5-methyl-1H-imidazol-4-yl)ethan-1-ol
17 1-(2-((3-chloro-4-fluorophenyl)((5-fluoro-6-methylpyridin-2-yl)amino)methyl)-1H-imidazol-4-yl)ethane-1,2-diol
18 3-(2-(bis(4-chloro-2-methoxyphenyl)methyl)-4-methyl-1H-imidazol-5-yl)pyrrolidin-3-ol
19 1-(2-((3-chloro-4-fluorophenyl)((6-(difluoromethyl)-5-fluoropyridin-2-yl)amino)methyl)-5-methyl-1H-imidazol-4-yl)ethane-1,2-diol
20 4-(2-(bis(4-chloro-2-methoxyphenyl)methyl)-4-methyl-1H-imidazol-5-yl)-1-methylpiperidin-4-ol
21 1-(2-((3-chloro-4-fluorophenyl)((6-(difluoromethyl)-5-fluoropyridin-2-yl)amino)methyl)-5-(methoxymethyl)-1H-imidazol-4-yl)ethane-1,2-diol
22 1-(2-(bis(4-chloro-2-methoxyphenyl)methyl)-5-(methoxymethyl)-1H-imidazol-4-yl)ethane-1,2-diol
23 3-amino-1-(2-(bis(4-chloro-2-methoxyphenyl)methyl)-4-methyl-1H-imidazol-5-yl)cyclobutan-1-ol
24 1-(2-(bis(3-chloro-4-fluorophenyl)methyl)-4-methyl-1H-imidazol-5-yl)ethan-1-amine
25 N-((4-(3-aminooxetan-3-yl)-1H-imidazol-2-yl)(3-chloro-4-fluorophenyl)methyl)-5-fluoro-6-methylpyridin-2-amine
26 1-(2-((3-chloro-4-fluorophenyl)((4,4-difluorocyclohexyl)oxy)methyl)-4-methyl-1H-imidazol-5-yl)ethan-1-amine
27 N-((4-(4-aminotetrahydro-2H-pyran-4-yl)-1H-imidazol-2-yl)(3-chloro-4-fluorophenyl)methyl)-5-fluoro-6-methylpyridin-2-amine
28 2-amino-2-(2-(bis(3-chloro-4-fluorophenyl)methyl)-5-methyl-1H-imidazol-4-yl)ethan-1-ol
29 2-(2-(bis(3-chloro-4-fluorophenyl)methyl)-4-methyl-1H-imidazol-5-yl)-2-methoxyethan-1-ol
30 1-(2-(bis(3-chloro-4-fluorophenyl)methyl)-4-methyl-1H-imidazol-5-yl)-2-methoxyethan-1-ol
31 2-(azetidin-1-yl)-2-(2-(bis(3-chloro-4-fluorophenyl)methyl)-5-methyl-1H-imidazol-4-yl)ethan-1-ol
32 1-(2-(bis(4,5-difluoro-2-methoxyphenyl)methyl)-5-methyl-1H-imidazol-4-yl)ethane-1,2-diol
33 2-amino-2-(2-((3-chloro-4-fluorophenyl)((5-fluoro-6-methylpyridin-2-yl)amino)methyl)-4-methyl-1H-imidazol-5-yl)ethan-1-ol
34 2-amino-1-(2-((3-chloro-4-fluorophenyl)((5-fluoro-6-methylpyridin-2-yl)amino)methyl)-4-methyl-1H-imidazol-5-yl)ethan-1-ol
35 azetidin-3-yl(2-(bis(3-chloro-4-fluorophenyl)methyl)-4-methyl-1H-imidazol-5-yl)methanol
36 5-(1-(azetidin-1-yl)-2-methoxyethyl)-2-(bis(3-chloro-4-fluorophenyl)methyl)-4-methyl-1H-imidazole
37 2-(azetidin-1-yl)-1-(2-(bis(3-chloro-4-fluorophenyl)methyl)-4-methyl-1H-imidazol-5-yl)ethan-1-ol
38 N-((5-(1-aminoethyl)-4-methyl-1H-imidazol-2-yl)(3-chloro-4-fluorophenyl)methyl)-5-fluoro-6-methylpyridin-2-amine
39 N-((5-(2-amino-1,3-dimethoxypropan-2-yl)-1H-imidazol-2-yl)(3-chloro-4-fluorophenyl)methyl)-5-fluoro-6-methylpyridin-2-amine
40 N-((5-((azetidin-3-yloxy)methyl)-4-methyl-1H-imidazol-2-yl)(3-chloro-4-fluorophenyl)methyl)-5-fluoro-6-methylpyridin-2-amine
41 4-(2-((3-chloro-4-fluorophenyl)((4-(trifluoromethyl)cyclohexyl)oxy)methyl)-5-methyl-1H-imidazol-4-yl)piperidin-4-ol
42 4-(2-((3-chloro-4-fluorophenyl)((4-(trifluoromethyl)cyclohexyl)oxy)methyl)-5-(methoxymethyl)-1H-imidazol-4-yl)piperidin-4-ol
43 4-(2-((3-chloro-4-fluorophenyl)((4-(trifluoromethyl)cyclohexyl)oxy)methyl)-5-(methoxymethyl)-1H-imidazol-4-yl)-1-methylpiperidin-4-ol
44 4-(2-((3-chloro-4-fluorophenyl)((4-(trifluoromethyl)cyclohexyl)oxy)methyl)-5-methyl-1H-imidazol-4-yl)-1-methylpiperidin-4-ol
45 2-(2-(bis(3-chloro-4-fluorophenyl)methyl)-5-methyl-1H-imidazol-4-yl)-2-(dimethylamino)ethan-1-ol
46 2-amino-2-(2-((3-chloro-4-fluorophenyl)((5-fluoro-6-methylpyridin-2-yl)amino)methyl)-5-methyl-1H-imidazol-4-yl)ethan-1-ol
47 N-((4-(1-amino-2-methoxyethyl)-5-methyl-1H-imidazol-2-yl)(3-chloro-4-fluorophenyl)methyl)-5-fluoro-6-methylpyridin-2-amine
48 2-(2-((3-chloro-4-fluorophenyl)((5-fluoro-6-methylpyridin-2-yl)amino)methyl)-5-methyl-1H-imidazol-4-yl)-2-(methylamino)ethan-1-ol
49 2-(2-((3-chloro-4-fluorophenyl)((5-fluoro-6-methylpyridin-2-yl)amino)methyl)-5-methyl-1H-imidazol-4-yl)-2-(dimethylamino)ethan-1-ol
50 N-((3-chloro-4-fluorophenyl)(4-(1-(dimethylamino)-2-methoxyethyl)-5-methyl-1H-imidazol-2-yl)methyl)-5-fluoro-6-methylpyridin-2-amine
51 N-((4-(1-amino-2,2-difluoroethyl)-5-methyl-1H-imidazol-2-yl)(3-chloro-4-fluorophenyl)methyl)-5-fluoro-6-methylpyridin-2-amine
52 N-((4-(1-amino-2-(difluoromethoxy)ethyl)-5-methyl-1H-imidazol-2-yl)(3-chloro-4-fluorophenyl)methyl)-5-fluoro-6-methylpyridin-2-amine
53 N-((4-(1-amino-2-(trifluoromethoxy)ethyl)-5-methyl-1H-imidazol-2-yl)(3-chloro-4-fluorophenyl)methyl)-5-fluoro-6-methylpyridin-2-amine
54 2-amino-2-(2-((3-chloro-4-fluorophenyl)((4-(trifluoromethyl)cyclohexyl)oxy)methyl)-5-methyl-1H-imidazol-4-yl)ethan-1-ol
55 1-(2-((3-chloro-4-fluorophenyl)((4-(trifluoromethyl)cyclohexyl)oxy)methyl)-5-methyl-1H-imidazol-4-yl)-2-methoxyethan-1-amine
56 2-(2-((3-chloro-4-fluorophenyl)((4-(trifluoromethyl)cyclohexyl)oxy)methyl)-5-methyl-1H-imidazol-4-yl)-2-(dimethylamino)ethan-1-ol
57 1-(2-((3-chloro-4-fluorophenyl)((4-(trifluoromethyl)cyclohexyl)oxy)methyl)-5-methyl-1H-imidazol-4-yl)-2-(difluoromethoxy)ethan-1-amine
58 1-(2-((3-chloro-4-fluorophenyl)((4-(trifluoromethyl)cyclohexyl)oxy)methyl)-5-methyl-1H-imidazol-4-yl)-2-(trifluoromethoxy)ethan-1-amine
59 N-((3-chloro-4-fluorophenyl)(4-(2-methoxy-1-(methylamino)ethyl)-5-methyl-1H-imidazol-2-yl)methyl)-5-fluoro-6-methylpyridin-2-amine
60 2-amino-2-(2-(bis(4-chloro-2-methoxyphenyl)methyl)-5-methyl-1H-imidazol-4-yl)ethan-1-ol
61 1-(2-(bis(4-chloro-2-methoxyphenyl)methyl)-5-methyl-1H-imidazol-4-yl)-2-methoxyethan-1-amine
62 2-(2-(bis(4-chloro-2-methoxyphenyl)methyl)-5-methyl-1H-imidazol-4-yl)-2-(methylamino)ethan-1-ol
63 2-(2-(bis(4-chloro-2-methoxyphenyl)methyl)-5-methyl-1H-imidazol-4yl)-2-(dimethylamino)ethan-1-ol
64 1-(2-(bis(4-chloro-2-methoxyphenyl)methyl)-5-methyl-1H-imidazol-4-yl)-2-(difluoromethoxy)ethan-1-amine
65 N-((3-chloro-4-fluorophenyl)(4-(2-(difluoromethoxy)-1-(methylamino)ethyl)-5-methyl-1H-imidazol-2-yl)methyl)-5-fluoro-6-methylpyridin-2-amine
66 2-amino-2-(2-(bis(3-chloro-4-fluorophenyl)methyl)-5-(difluoromethyl)-1H-imidazol-4-yl)ethan-1-ol
67 2-amino-2-(2-(bis(3-chloro-4-fluorophenyl)methyl)-5-(trifluoromethyl)-1H-imidazol-4-yl)ethan-1-ol
68 2-amino-2-(2-(bis(3-chloro-4-fluorophenyl)methyl)-5-chloro-1H-imidazol-4-yl)ethan-1-ol
69 2-amino-2-(2-((3-chloro-4-fluorophenyl)((5-fluoro-6-methylpyridin-2-yl)amino)methyl)-5-methyl-1H-imidazol-4-yl)propan-1-ol
70 4-(1-amino-2-hydroxyethyl)-2-(bis(3-chloro-4-fluorophenyl)methyl)-1H-imidazole-5-carbonitrile
and the physiologically acceptable salts thereof.

In a preferred embodiment, the compound according to the invention is an inhibitor of Na_{V}1.8. In the sense of the invention, the term "inhibitor of Na_{V}1.8" preferably means that the respective compound exhibits in a patch clamp assay an IC50 value on Na_{V}1.8 of at most 10 µM (10·10⁻⁶ mol/L); more preferably at most 1 µM; still more preferably at most 500 nM (10⁻⁹ mol/L); even more preferably at most 100 nM; and most preferably at most 10 nM.

A preferred assay for testing compounds for their potency and method for determining an IC50 on Na_{V}1.8 is described in the experimental part down below.

In a preferred embodiment, the compound according to the invention is a selective inhibitor of Na_{V}1.8. In the sense of the invention, the term "selective inhibitor of Na_{V}1.8" preferably means that the respective compound preferably does not exhibit any inhibitory activity on Na_{V}1.1, Na_{V}1.2, Na_{V}1.4, Na_{V}1.5 and Na_{V}1.6. The skilled artisan knows suitable ways to determine whether a compound exhibits inhibitory effects on any of Na_{V}1.1, Na_{V}1.2, Na_{V}1.4, Na_{V}1.5 and Na_{V}1.6.

The invention therefore relates to a compound according to the invention for use in the inhibition of Na_{V}1.8.

Therefore, another aspect of the invention relates to a compound according to the invention for use in the treatment of pain. Still another aspect of the invention relates to a method of treatment of pain; comprising the administration of a therapeutically effective amount of a compound according to the invention to a subject in need thereof, preferably a human.

A further aspect of the invention relates to a compound according to the invention as medicament.

Another aspect of the invention relates to a pharmaceutical dosage form comprising a compound according to the invention. Preferably, the pharmaceutical dosage form comprises a compound according to the invention and one or more pharmaceutical excipients such as physiologically acceptable carriers, additives and/or auxiliary substances; and optionally one or more further pharmacologically active ingredient. Examples of suitable physiologically acceptable carriers, additives and/or auxiliary substances are fillers, solvents, diluents, colorings and/or binders. These substances are known to the person skilled in the art (see H. P. Fiedler, Lexikon der Hilfsstoffe fur Pharmazie, Kosmetik und angrenzende Gebiete, Editio Cantor Aulendoff).

The pharmaceutical dosage form according to the invention is preferably for systemic, topical or local administration, preferably for oral administration. Therefore, the pharmaceutical dosage form can be in form of a liquid, semisolid or solid, e.g. in the form of injection solutions, drops, juices, syrups, sprays, suspensions, tablets, patches, films, capsules, plasters, suppositories, ointments, creams, lotions, gels, emulsions, aerosols or in multiparticulate form, for example in the form of pellets or granules, if appropriate pressed into tablets, decanted in capsules or suspended in a liquid, and can also be administered as such.

The pharmaceutical dosage form according to the invention is preferably prepared with the aid of conventional means, devices, methods and processes known in the art. The amount of the compound according to the invention to be administered to the patient may vary and is e.g. dependent on the patient's weight or age and also on the type of administration, the indication and the severity of the disorder. Preferably 0.001 to 100 mg/kg, more preferably 0.05 to 75 mg/kg, most preferably 0.05 to 50 mg of a compound according to the invention are administered per kg of the patient's body weight.

Therefore, another aspect of the invention relates to the pharmaceutical dosage form according to the invention for use in the treatment of pain. Still another aspect of the invention relates to a method of treatment of pain; comprising the administration of a pharmaceutical dosage form according to the invention to a subject in need thereof, preferably a human.

### General synthesis schemes

Examples of the invention can be synthesized preferentially according to the general synthetic schemes below or using chemistry known to the person skilled in the art.

Protection of imidazoles can lead to two different regioisomers. Both regioisomers can generally undergo similar transformation in similar set of conditions. In the following, only one of the regiosiomers is indicated for clarity. Deprotection of the two regioisomers will lead to the same final compounds.

As illustrated in Scheme 1 for compounds with L=bond, one equivalent of compounds of the general formula (A) can be treated with two equivalents of metal-organic compounds of the general formula (B) (wherein A=B, R2=R3 and x=y) to afford compounds of general formula (C). Preferred metal-organic compounds include Grignard and organolithium reagents. Compounds of the general formula (C) can be converted into compounds of the general formula (D) with reactions known to the person skilled in the art. Preferred transformations include reductions to get compounds of the general formula (D), preferred reagents include tin (II)chloride in acetic media.

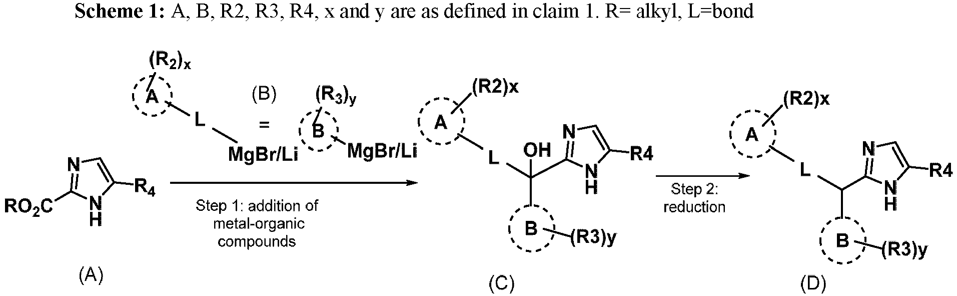

Aldehydes of the general formula (E), protected imidazoles of the general formula (F) and N,N-bis(propan-2-yl)carbamoyl chloride (G) in the presence of base (preferred bases include DIPEA) can be reacted in a multicomponent reaction to get compounds of the general formula (H). Acid-catalyzed displacement between compounds of the general formula (H) and alcohols (L=O) of the general formula (J) or amines (L=NH) of the general formula (J) yields compounds of the general formula (D). Preferred conditions for this displacement include the use of BF₃·Et₂O in combination with TFA, which in some cases leads to concomitant deprotection. In some cases, intermediate protection and later deprotection of the imidazole may be warranted, preferred protecting groups include SEM.

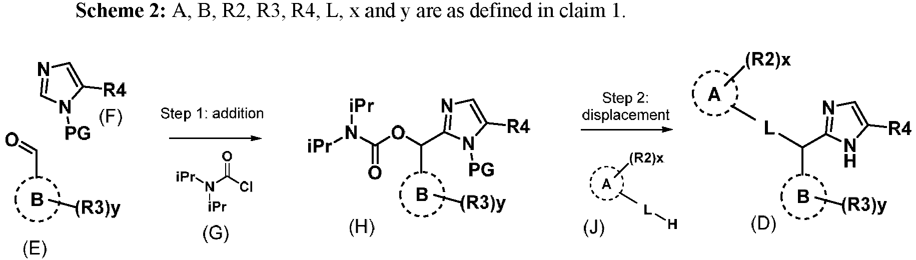

As illustrated in Scheme 3, compounds of the general formula (D) can be halogenated, preferentially iodinated using I₂ or NIS to obtain compounds of the general formula (K). These compounds can be used to install a variety of functionalized moieties to yield compounds of the general formula (I) via one step or multi step procedures. These include the addition of suitable aldehydes, ketones, sulfinyl aldimines or sulfinyl ketimines via methods known in the art. Alkenylation followed by commonly used dihydroxylation may provide access to vicinal diols as compounds of the invention or intermediates. Subsequent functional group interconversion employing strategies as alkylation or transformation into a leaving group and replacement by nucleophilic substitution with suitable nucleophiles allows for a variety of residues R6 and R7 according to general formula (I). Certain transformations require the intermediate protection and later deprotection of the imidazole, preferred protecting groups include SEM. Certain transformation require the use of intermediate protecting groups on amines, such as Boc. Protection and deprotection of both SEM and Boc are known in the art. Alternatively, a variation of reaction sequences illustrated in Schemes 1-3 may be employed to provide access to compounds of the invention.

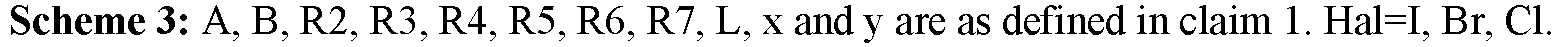

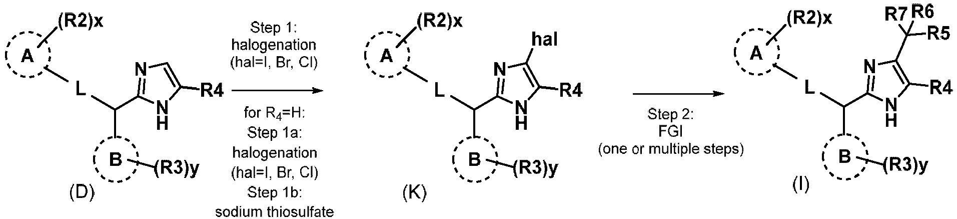

### List of abbreviations

ABPR= automated back pressure regulator, ACN= acetonitrile, BINAP= [2,2'-Bis(diphenylphosphino)-1,1'-binaphthyl], Boc= tert-butyloxycarbonyl, dba= dibenzylideneacetone, DCM= dichloromethane, DIBAL= diisobutylaluminium hydride, DIPEA= N,N-diisopropylethylamine, DMF= dimethylformamide, DMSO= dimethyl sulfoxide, dppf= 1,1'-bis(diphenylphosphino)ferrocene, EtOAc/EA=ethyl acetate, FGI=functional group interconversion, Hex=hexane/hexanes, HPLC= high-performance liquid chromatography, iPrNH2 = Isopropylamine, iPrOH = Isopropanol, LCMS= liquid chromatography-mass spectrometry, MeOH = Methanol, nBuLi = n-Butyl lithium, NIS= N-iodosuccinimide, NMO = N-Methylmorpholine-N-oxide, NMR= nuclear magnetic resonance, PG=protecting group, Rt=retention time, SEM= 2-(trimethylsilyl)ethoxymethyl, SFC= supercritical fluid chromatography, TBAF= tetra-n-butylammonium fluoride, TEA= triethylamine, TFA= trifluoroacetic acid, THF=tetrahydrofurane.

### LCMS Methods

**Method C:** Applied Biosystems LCMS/MS API 2000 with HPLC Shimadzu Prominence; Column: Xbridge (C18 4.6 × 50 mm, 5 micron); Column Temperature: 25 °C.

### Solvent A: Buffer 10 mM NH₄OAc in water and solvent B: ACN; Flow rate: 1.2 mL/min

Gradient: mobile phase from 90% [buffer] and 10% [ACN] to 70% [buffer] and 30% [ACN] in 1.5 min, further to 10% [buffer] and 90% [ACN] in 3.0 min, held this mobile phase composition to 4 min and finally back to initial condition in 5 min).

**Method G:** Water Acquity H Class UPLC attached with Waters SQD 2 mass spectrometer; Column: YMC TRIART (2.1 × 33 mm); Column Temperature: 50 °C.

### Solvent A: 5 mM formic acid in water and Solvent B: ACN; Flow rate: 1.5 mL/min

Gradient: mobile phase from 98% [buffer] and 2% [ACN] held for 0.75 min, then to 90% [buffer] and 10% [ACN] in 1.00 min, further to 2% [buffer] and 98% [ACN] in 2.0 min, held this mobile phase composition to 2.25 min and finally back to initial condition in 2.90 min and held this composition up to 3.00 min.

**Method S:** Water Acquity H Class UPLC attached with Waters SQD 2 mass spectrometer; Column: Waters Acquity UPLC BEH C8 (2.1 × 50 mm, 1.7 micron); Column Temperature: 50 °C.

Solvent A: 0.05% Formic acid in water and Solvent B: 0.05% HCOOH in ACN: Water (90:10); Flow rate: 0.8 mL/min.

Gradient: mobile phase: 5% [0.05% HCOOH in water] and 95% [0.05% HCOOH in ACN: Water (90:10)] held for 0.75 min, then to 75% [0.05% HCOOH in water] and 25% [0.05% HCOOH in ACN: Water (90:10) ] in 1.5 min, further to 5% [0.05% HCOOH in water] and 95% [0.05% HCOOH in ACN: Water (90:10) ] in 3.00 min, held this mobile phase composition up to 4.00 min and finally back to initial condition in 4.50 min and held this composition up to 5.10 min.

**Method U:** Water Acquity H Class UPLC attached with Waters SQD 2 mass spectrometer; Column: Xbridge C18 (3 ×50 mm, 3.5 micron); Column Temperature: 50°C.

Solvent A: 5 mM NH₄OAc in water and Solvent B: 5 mM NH₄OAc in ACN: Water (90:10); Flow rate: 1.20 ml/min.

Gradient: mobile phase: 95% [5 mM NH₄OAc in water] and 5% [5 mM NH₄OAc in ACN: Water (90:10)] held for 0.75 min, then to 70% [5 mM NH₄OAc in water] and 30% [5 mM NH₄OAc in ACN: Water (90:10) ] in 1.00 min, and finally 2% [5 mM NH₄OAc in water] and 98% [5 mM NH₄OAc in ACN: Water (90:10) ] in 2.00 min held this mobile phase composition up to 2.50 min and finally back to initial condition in 2.75 min and held this composition up to 3.0 min.

**Method V:** Water Acquity H Class UPLC attached with Waters SQD 2 mass spectrometer; Column: Xbridge C18 (3 ×50 mm, 3.5 micron); Column Temperature: 50°C.

Solvent A: 5 mM NH₄OAc in water and Solvent B: 5 mM NH₄OAc in ACN: Water (90:10); Flow rate: 1.20 ml/min.

Gradient: mobile phase: 95% [10 mM NH4OAc in water] and 5% [10 mM NH4OAc in ACN: Water (90:10)] held for 0.75 min, then to 85% [10 mM NH4OAc in water] and 15% [10 mM NH4OAc in ACN: Water (90:10) ] in 1.25 min, further to 30% [10 mM NH4OAc in water] and 70% [10 mM NH4OAc in ACN: Water (90:10) ] in 2.50 min , then 2% [10 mM NH4OAc in water] and 98% [10 mM NH4OAc in ACN: Water (90:10) ] in 3.75 min, held this mobile phase composition up to 4.25 min and finally back to initial condition in 4.50 min and held this composition up to 5.10 min.

### Intermediate 1

### 2-(bis(3-chloro-4-fluorophenyl)methyl)-5-iodo-4-methyl-1H-imidazole

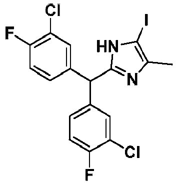

Step 1: Mg (3.2 g, 130 mmol) was taken up in a 2-necked flask equipped with a reflux condenser and dried under argon atmosphere. THF (30 mL), 4-bromo-2-chloro-1-fluorobenzene (15.8 mL, 130 mmol) and DIBAL (1M in toluene) (2.0 mL) were added to the mixture sequentially at ambient temperature. The reaction mixture was stirred for 1 h. To this mixture, a solution of ethyl 4-methyl-1H-imidazole-2-carboxylate (4.0 g, 26 mmol) in THF (170 mL) was added at 0 °C and the reaction mixture was heated to 80 °C for 5 h. The reaction mixture was cooled to rt and quenched with ammonium chloride solution. The aqueous layer was filtered through a celite bed and extracted with ethyl acetate (3 x 200 mL). The combined organic layers were washed with brine, dried over Na₂SO₄ and concentrated under reduced pressure to get the crude product which was purified by column chromatography (SiO₂, 0-60% EtOAc/Hex) to yield bis(3-chloro-4-fluorophenyl)(5-methyl-1H-imidazol-2-yl)methanol (7.1 g, 74%), LCMS: m/z [M+H]⁺ = 369.

Step2: To a solution of bis(3-chloro-4-fluorophenyl)(5-methyl-1H-imidazol-2-yl)methanol (7.0 g, 19 mmol) in acetic acid (100 mL) were added SnCl₂ (21.6 g, 114 mmol) and conc. HCl (21 mL) at 0 °C. The reaction mixture was stirred at 120 °C under reflux conditions for 16 h. The reaction mixture was cooled to ambient temperature and concentrated under reduced pressure. The resulting crude residue was basified with Na₂CO₃ solution. The aqueous layer was filtered through a celite bed and the filtrate was extracted with ethyl acetate (3 × 200 mL). The combined organic layers were washed with brine, dried over Na₂SO₄ and concentrated under reduced pressure to get the crude product which was washed with 10% ethyl acetate in hexane to obtain 2-[bis(3-chloro-4-fluorophenyl)methyl]-5-methyl-1H-imidazole (5.2 g, 77%), LCMS: m/z [M+H]⁺ = 353.

Step 3: To a solution of 2-[bis(3-chloro-4-fluorophenyl)methyl]-5-methyl-1H-imidazole (2.5 g, 7.1 mmol) in DMF (30 mL) were added KOH (0.6 g, 10.7 mmol) and I₂ (2.4 g, 9.2 mmol) at 0 °C. The reaction mixture was then stirred at ambient temperature for 1 h. The reaction mixture was quenched with sodium thiosulfate solution, the aqueous layer was extracted with ethyl acetate (2 × 150 mL). The combined organic layers were washed with brine, dried over Na₂SO₄ and evaporated under reduced pressure to get the crude product which was purified by column chromatography (SiO₂, 0-40% EtOAc/Hex) to yield the title compound (2.2 g, 64%), LCMS: m/z [M+H]⁺ = 479, Rt=2.46 min.

### Intermediate 2

### 2-[bis(3-chloro-4-fluorophenyl)methyl]-4-iodo-5-methyl-1-{[2-(trimethylsilyl)ethoxy]methyl}-1H-imidazole

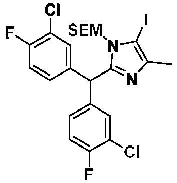

To a stirred solution of 2-[bis(3-chloro-4-fluorophenyl)methyl]-4-iodo-5-methyl-1H-imidazole (7.3 g, 15.2 mmol) in DCM (80 mL) were added DIPEA (4.0 mL, 22.9 mmol) and 2-(trimethylsilyl)ethoxymethyl chloride (3.2 mL, 18.3 mmol) at 0 °C. The reaction mixture was stirred at room temperature for 16 h and subsequently diluted with water and extracted with DCM (3 × 100 mL). The organic layer was washed with brine (75 mL) and dried over Na₂SO₄. The solvent was evaporated under reduced pressure. The crude product was purified by column chromatography (SiO₂; 0-20% EtOAc/Hex) to yield the title compound (6.4 g, 64%). LCMS: m/z [M+H]⁺ = 609, Rt=4.19 min (method S)

### Intermediate 3

### N-[(3-chloro-4-fluorophenyl)(4-iodo-5-methyl-1-{[2-(trimethylsilyl)ethoxy]methyl}-1H-imidazol-2-yl)methyl]-5-fluoro-6-methylpyridin-2-amine

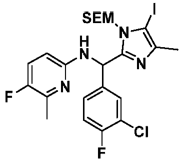

Step 1: To a stirred solution of 4-iodo-5-methyl-1H-imidazole (15.0 g, 72.1 mmol) in toluene (100 mL) was added DIPEA (25.0 mL, 144.2 mmol) at 0 °C and stirred for 5 minutes. Then SEM-Cl (15.5 mL, 86.5 mmol) was added dropwise to reaction mixture at 0 °C. The reaction mixture was stirred at 90°C for 16 h. Subsequently the reaction mixture was quenched with saturated NH₄Cl solution, diluted with EtOAc (250 mL) and washed with H₂O (100 mL) and brine (100 mL). The organic extract was dried over anhydrous Na₂SO₄, filtered and concentrated under reduced pressure. The crude product was purified by flash chromatography (SiO₂; 0-30% EtOAc/Hex) to afford 4-iodo-5-methyl-1-{[2-(trimethylsilyl)ethoxy]methyl}-1H-imidazole as single regio isomer (3.48 g, 14%). LCMS: m/z [M+H]⁺ = 339.

Step 2: To a stirred solution of 4-iodo-5-methyl-1-((2-(trimethylsilyl)ethoxy)methyl)-1H-imidazole (3.6 g, 10.6 mmol) in ACN (150 mL) were added 3-chloro-4-fluorobenzaldehyde (1.85 g, 11.7 mmol), N,N-bis(propan-2-yl)carbamoyl chloride (2.0 g, 12.7 mmol) and DIPEA (5.6 mL, 31.8 mmol) at ambient temperature and the reaction mixture was stirred at 80 °C for 16 h. After the reaction was completed, the reaction mixture was concentrated under reduced pressure. The crude material was diluted with EtOAc (150 mL), washed with H₂O (2 × 50 mL) and brine (50 mL). The organic layer was dried over anhydrous Na₂SO₄, filtered and concentrated under reduced pressure. Purification by flash chromatography (SiO₂; 0-50% EtOAc/Hex) to afforded (3-chloro-4-fluorophenyl)(4-iodo-5-methyl-1-{[2-(trimethylsilyl)ethoxy]methyl}-1H-imidazol-2-yl)methyl N,N-bis(propan-2-yl)carbamate (3.67 g, 55%). LCMS: m/z [M+H]⁺ = 624.

Step 3: To a stirred solution of (3-chloro-4-fluorophenyl)(4-iodo-5-methyl-1-{[2-(trimethylsilyl)ethoxy]methyl}-1H-imidazol-2-yl)methyl N,N-bis(propan-2-yl)carbamate (3.67 g, 5.9 mmol) and 5-fluoro-6-methylpyridin-2-amine (1.85 g, 14.7 mmol) in DCM (30 ml) were added BF₃.Et₂O (1.0 ml, 8.8 mmol) and TFA (2.0 ml, 26 mmol) at 0°C and the reaction was stirred at ambient temperature for 16 hours. After completion of the reaction the mixture was quenched with NaHCO₃ (40 mL) and extracted with EtOAc (2 × 30 mL). The combined organic layer was washed with brine (30 mL), dried over anhydrous Na₂SO₄ and evaporated under reduced pressure to get crude product. The crude material was purified by flash chromatography (SiO₂; 0-60% EtOAc/Hex) to afford N-[(3-chloro-4-fluorophenyl)(4-iodo-5-methyl-1-{[2-(trimethylsilyl)ethoxy]methyl}-1H-imidazol-2-yl)methyl]-5-fluoro-6-methylpyridin-2-amine (3.08 g, 86%). LCMS: m/z [M+H]⁺ = 605, Rt = 4.07 min (method S).

The following intermediates may be prepared from the appropriate starting materials and reagents using similar methods.

| Intermediate | Structure | Analytical Data |
|---|---|---|
| **4** | 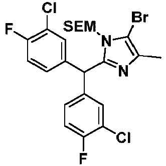 | LCMS: m/z [M+H]⁺ = 561, Rₜ=2.73; 2.89 min (method C) |
| **5** | 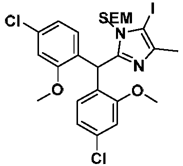 | LCMS: m/z [M+H]⁺ = 633, Rₜ=4.28 min (method U) |
| **6** | 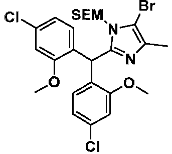 | LCMS: m/z [M+H]⁺ = 585, Rₜ=3.74 min (method S) |
| **7** | 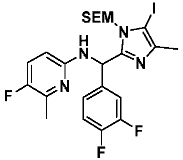 | LCMS: m/z [M+H]⁺ = 589, Rₜ=4.53 min (method C) |
| **8** | 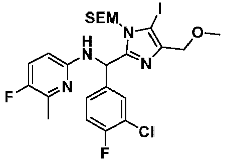 | LCMS: m/z [M+H]⁺ = 635, Rₜ=3.94 min (method C) |
| **9** | 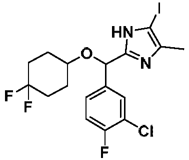 | LCMS: m/z [M+H]⁺ = 485, Rₜ=2.68 min (method G) |
| **10** | 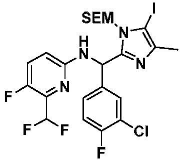 | LCMS: m/z [M+H]⁺ = 641, Rₜ=2.47 min (method C) |
| **11** | 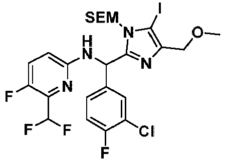 | LCMS: m/z [M+H]⁺ = 671, Rₜ=3.80, 3.93 min (method S) |

### Example 1a, 1b

### 1-(2-(bis(3-chloro-4-fluorophenyl)methyl)-5-methyl-1H-imidazol-4-yl)ethane-1,2-diol

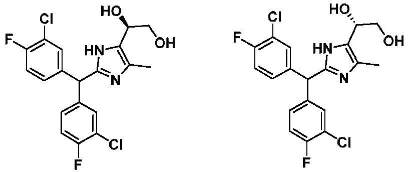

Step 1: A stirred solution of 2-[bis(3-chloro-4-fluorophenyl) methyl]-5-iodo-4-methyl-1-{ [2-(trimethylsilyl) ethoxy] methyl}-1H-imidazole (intermediate 2, 1.0 g, 1.64 mmol) in 1,4-dioxane (9 mL) and H₂O (3 mL) was degassed with argon for 2 minutes in a sealed tube. Then ethenyltrifluoro-λ⁴-borane potassium (0.44 g, 3.3 mmol), K₂CO₃ (0.68 g, 4.9 mmol) and Pd(dppf)Cl₂ (0.12 g, 0.16 mmol) were added. The resulting mixture was stirred at 80 °C for 16 h, then diluted with EtOAc (50 mL) and washed with H₂O (20 mL) and brine (20 mL). The organic extract was dried over anhydrous Na₂SO₄, filtered and concentrated under reduced pressure. The crude product was purified by flash chromatography (SiO₂, 0-30% EtOAc/Hex) to afford 2-[bis(3-chloro-4-fluorophenyl)methyl]-5-ethenyl-4-methyl-1-{[2-(trimethylsilyl)ethoxy]methyl}-1H-imidazole (0.56 g, 67%). LCMS m/z = 509 [M+H]⁺.

Step 2: To a stirred solution of 2-[bis(3-chloro-4-fluorophenyl)methyl]-5-ethenyl-4-methyl-1-{[2-(trimethylsilyl)ethoxy]methyl}-1H-imidazole (0.20 g, 0.39 mmol) in Acetone (4 mL) and H₂O (0.5 mL) were added NMO (0.092 g, 0.785 mmol), OsO₄ (4% in water) (0.250 mL, 0.039 mmol). The resulting mixture was stirred at ambient temperature for 16 h and subsequently quenched with Na₂S₂O₃ solution and extracted with EtOAc (20 mL) and washed with H₂O (10 mL) and brine (10 mL). The combined organic layer was dried over anhydrous Na₂SO₄, filtered and concentrated under reduced pressure. Th crude material was purified by flash chromatography (SiO₂, 0-100% EtOAc:EtOH (3:1)/Hex) to afford 1-{2-[bis(3-chloro-4-fluorophenyl)methyl]-4-methyl-1-{[2-(trimethylsilyl)ethoxy]methyl}-1H-imidazol-5-yl}ethane-1,2-diol (0.20 g, 93 %). LCMS m/z = 543 [M+H]⁺.

Step 3: To a stirred solution of 1-{2-[bis(3-chloro-4-fluorophenyl) methyl]-4-methyl-1-{[2(trimethylsilyl)ethoxy] methyl}-1H-imidazol-5-yl} ethane-1,2-diol (0.22 g, 0.41 mmol) in DCM (4 ml) was added TFA (4 ml) at 0°C and the reaction mixture was stirred at ambient temperature for 16h. The solvent was evaporated to get the crude product, which was purified by reverse-phase prep HPLC followed by the chiral separation using chiral SFC to yield the title compound.

LCMS m/z = 413 [M+H]⁺; ¹H-NMR (400 MHz , 100°C, DMSO-d₆): δ 11.32 (s, 1H), 7.52 (m, 2H), 7.32 (m, 4H), 5.50 (s, 1H), 4.79 (bs, 1H), 4.50 (bs, 1H), 4.27-4.24 (bs, 2H), 3.56 (s, 2H), 2.13 (s, 3H) .Chiral SFC: column: Chiralcel OX-H (21.1 x 250 mm )5 µm; co-solvent: 0.3% iPrNH₂ in iPrOH, flow: 3 mL/min; % of co-solvent: 30%; ABPR: 110 bar: 35 °C; **Example 1a:** Yield: 0.025 g, (14%). Rt = 2.37 min (first eluting stereoisomer with unknown absolute configuration). **Example 1b:** Yield: 0.021 g, (12%). Rt = 2.89 min (second eluting stereoisomer with unknown absolute configuration).

### Example 8a, 8b, 8c

### 1-(2-((3-chloro-4-fluorophenyl)((5-fluoro-6-methylpyridin-2-yl)amino)methyl)-5-methyl-1H-imidazol-4-yl)ethane-1,2-diol

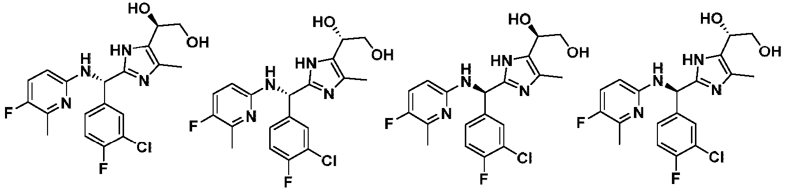

Step 1: To a stirred solution of N-[(3-chloro-4-fluorophenyl)(4-iodo-5-methyl-1-{ [2-(trimethylsilyl)ethoxy]methyl}-1H-imidazol-2-yl)methyl]-5-fluoro-6-methylpyridin-2-amine (intermediate 3, 3.08 g, 5.01 mmol) in 1,4-dioxane (18 mL) and H₂O (6 mL) was degassed with argon for 2 minutes in a sealed tube. Then ethenyltri-fluoro-λ⁴-borane potassium (1.37 g, 10.2 mmol), K₂CO₃ (2.11 g, 15.3 mmol) and Pd(dppf)Cl₂ (0.37 g, 0.51 mmol) were added. The resulting mixture was stirred at 80°C for 16 h then diluted with EtOAc (70 mL) and washed with H₂O (20 mL) and brine (20 mL). The organic extract was dried over anhydrous Na₂SO₄, filtered (SiO₂; 0-30% EtOAc/Hex) to afford N-[(3-chloro-4-fluorophenyl)(4-ethenyl-5-methyl-1-{[2-(trimethylsilyl)ethoxy]methyl}-1H-imidazol-2-yl)methyl]-5-fluoro-6-methylpyridin-2-amine (1.86 g, 72%). LCMS m/z = 505 [M+H]⁺.

Step 2: To a stirred solution of N-[(3-chloro-4-fluorophenyl)(4-ethenyl-5-methyl-1-{[2-(trimethylsilyl)ethoxy]methyl}-1H-imidazol-2-yl)methyl]-5-fluoro-6-methylpyridin-2-amine (1.86 g, 3.68 mmol) in THF (5 mL) were added ADmix alpha (5.19 g, 6.67 mmol), ADmix beta (5.19 g, 6.67 mmol) t-BuOH:H₂O(1:1).The resulting mixture was stirred at ambient temperature for 16 h, then quenched with sodium sulfite solution and extracted with EtOAc (70 mL) and washed with H₂O (20 mL) and brine (20 mL). The organic extract was dried over anhydrous Na₂SO₄, filtered and concentrated under reduced pressure. The crude product was purified by flash chromatography (SiO_{2,} 0-100% EtOAc:EtOH (3:1)/Hex) to afford 1-{2-[(3-chloro-4-fluorophenyl)[(5-fluoro-6-methylpyridin-2-yl)amino]methyl]-4-methyl-1-{[2-(trimethylsilyl)ethoxy]methyl}-1H-imidazol-5-yl}ethane-1,2-diol (1.2 g, 60 %). LCMS m/z = 539 [M+H]⁺.

Chiral Separation by chiral SFC afforded Peak-1 (mixture of 1^{st} & 2^{nd} eluting isomers, 0.58 g), Peak-2 (3^{rd} eluting isomer, 0.15 g) and Peak-3 (4th eluting isomer), 0.16 g).
Step 3: To a stirred solution of Peak-2 (0.121 g, 0.22 mmol) in DCM (2.5 ml) was added TFA (2.5 ml) at 0°C and the reaction mixture was stirred at ambient temperature for 16h. The reaction mixture was concentrated under reduced pressure and purified by prep-HPLC to afford the title compound as example 8a (0.055 g, 59 %). Peak 1 and peak 3 were processed in analogy.

Example 8a (from peak 2, unknown absolute configuration): LCMS m/z = 409 [M+H]⁺; ¹H-NMR (400 MHz , 100°C, DMSO-d₆): δ 11.33 (bs, 1H), 7.59 (d, J = 6.8 Hz, 1H), 7.40 (s, 1H), 7.29-7.21 (m, 2H), 6.87 (s, 1H), 6.50 (d, J = 8.0 Hz, 1H), 6.14 (s, 1H), 4.79 (bs, 1H), 4.54 (bs, 1H), 4.23 (bs, 2H), 3.56 (bs, 2H), 2.24 (s, 3H), 2.12 (bs, 3H). Chiral SFC: column: CHIRALPAK IC (4.6 × 250 mm), 5 µm; co-solvent: 0.3% iPrNH₂ in iPrOH, flow: 4 mL/min; % of co-solvent: 40%; ABPR: 100 bar: 35 °C; Rt = 1.46 min.

**Example 8b** (from peak 3, unknown absolute configuration): LCMS m/z = 409 [M+H]⁺; ¹H-NMR (400 MHz , 100°C, DMSO-d₆): δ 11.33-11.24 (m, 1H), 7.59 (d, J = 6.8 Hz, 1H), 7.40 (s, 1H), 7.29-7.20 (m, 2H), 6.85 (s, 1H), 6.50 (d, J = 8.0 Hz, 1H), 6.13 (s, 1H), 4.74 (bs, 1H), 4.58-4.50 (m, 1H), 4.30 (bs, 1H) , 4.17 (bs, 1H) 3.57 (m, 2H), 2.24 (s, 3H), 2.12 (m,3H). Chiral SFC: column: CHIRALPAK IC (4.6 × 250 mm), 5 µm; co-solvent: 0.3% iPrNH₂ in iPrOH, flow: 4 mL/min; % of co-solvent: 40%; ABPR: 100 bar: 35 °C; Rt = 1.47 min.

**Example 8c** (mixture of 2 stereoisomers from peak 1 with unknown absolute configuration): LCMS m/z = 409 [M+H]⁺; ¹H-NMR (400 MHz , 100°C, DMSO-d₆): δ 11.217 (bs, 1H), 7.59 (d, J = 6.8 Hz, 1H), 7.40 (s, 1H), 7.28-7.20 (m, 2H), 6.83 (s, 1H), 6.50 (d, J = 8.8 Hz, 1H), 6.14 (s, 1H), 4.54 (s, 3H), 3.55 (s, 2H), 2.32-2.24 (m, 3H), 2.12 (s, 3H). Chiral SFC: column: CHIRALPAK IC (4.6 × 250 mm), 5 µm; co-solvent: 0.5% iPrNH₂ in iPrOH, flow: 4 mL/min; % of co-solvent: 40%; ABPR: 100 bar: 35 °C; Rt = 1.21 min.

The following examples may be prepared from the appropriate intermediates and reagents using similar methods to those described for examples 1 and 8. Single stereoisomers may be obtained by chiral separation, either on intermediate or final stage. The absolute configuration remains unknown and cannot be deduced from the order of representations.

| Ex. | Name/Structure | Analytical Data |
|---|---|---|
| **4a, 4b** | 1-(2-((3,4-difluorophenyl)((5-fluoro-6-methylpyridin-2-yl)amino)methyl)-5-methyl-1H-imidazol-4-yl)ethane-1,2-diol | LCMS m/z = 393 [M+H]⁺; Chiral HPLC: column: Chiralpak IC (4.6 x 250 mm) 5 µm, mobile phase: Hexane/EtOH/iPrNH₂ 70/30/0.1, flow rate: 1.0 mL/min; **4a:** Rt = 1.66 min (first eluting peak, mixture of 2 stereoisomeres), **4b:** Rt = 1.70 min (second eluting peak, mixture of 2 stereoisomers). |
| | 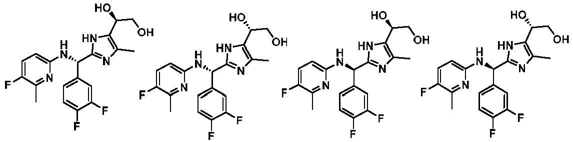 | |
| **5a, 5b** | 1-(2-((3-chloro-4-fluorophenyl)((4,4-difluorocyclohexyl)oxy)methyl)-5-methyl-1H-imidazol-4-yl)ethane-1,2-diol | LCMS m/z = 409 [M+H]⁺;Chiral SFC: column: (R,R)-WHELK-01 3.5um, co-solvent: 0.5% iPrNH₂ in iPrOH; flow: 4 mL/min; % of co-solvent: 30%; ABPR: 1450 psi; T: 35 °C; **5a:** Rt = 1.07 min (first eluting peak, mixture of 2 stereoisomeres), **5b:** Rt = 1.28 min (second eluting peak, mixture of 2 stereoisomers). |
| | 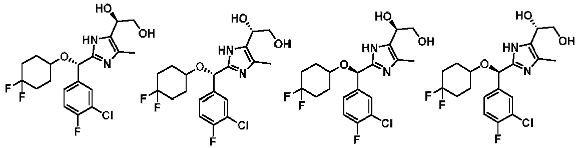 | |
| **6a, 6b, 6c** | 1-(2-((3-chloro-4-fluorophenyl)((5-fluoro-6-methylpyridin-2-yl)amino)methyl)-5-(methoxymethyl)-1H-imidazol-4-yl)ethane-1,2-diol | LCMS m/z = 439 [M+H]⁺;Chiral SFC: column: CHIRALPAK IC (4.6 × 150 mm), 5 µm, co-solvent: 0.5% iPrNH₂ in iPrOH; flow: 3 mL/min; % of co-solvent: 20%; ABPR: 1500 psi; T: 35 °C; **6a:** Rt = 3.47 min (first eluting stereoisomer), **6b:** Rt = 3.96 min (second eluting stereoisomer), **6c:** third eluting peak (mixture of 2 stereoisomers). |
| | 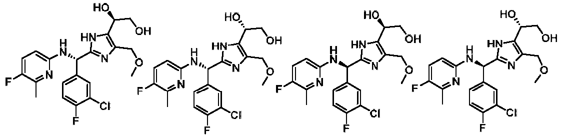 | |
| **14a, 14b** | 1-(2-(bis(4-chloro-2-methoxyphenyl)methyl)-5-methyl-1H-imid- | LCMS m/z = 437 [M+H]⁺;Chiral SFC: column (R,R) Whelk O-1 (4.6 × 250 mm), 5µm, co-solvent: 0.5% iPrNH2 in iPrOH; flow: 3 mL/min; % of co-solvent: 25%; ABPR: 100 bar; T: 35 °C; **14a:** Rt = 2.72 min (first eluting stereoisomer) , **14b:** Rt = 3.11 min (second eluting stereoisomer) |
| | 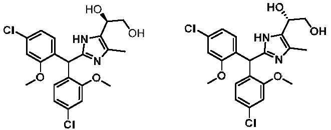 azol-4-yl)ethane-1,2-diol | |
| **19a, 19b** | 1-(2-((3-chloro-4-fluorophenyl)((6-(difluoromethyl)-5-fluoro-pyridin-2-yl)amino)methyl)-5-methyl-1H-imidazol-4-yl)ethane-1,2-diol | **19a** (mixture of stereoisomers, first eluting): LCMS m/z = 445 [M+H]+; Rt = 2.38 min (method S) |
| | 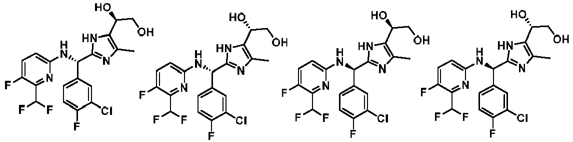 | **19b** (mixture of stereoisomers, second eluting): LCMS m/z = 445 [M+H]+; Rt = 2.40 min (method S) |
| **21a, 21b** | 1-(2-((3-chloro-4-fluorophenyl)((6-(difluoromethyl)-5-fluoro-pyridin-2-yl)amino)methyl)-5-(methoxymethyl)-1H-imidazol-4-yl)ethane-1,2-diol | LCMS m/z = 475 [M+H]+;Chiral SFC: column: C-AMYLOSE A (4.6 × 150 mm), 5 µm, co-solvent: 0.3% iPrNH2 in ACN/MeOH (70:30); flow: 3 mL/min; % of co-solvent: 25%; ABPR: 1500 psi; T: 35 °C; **21a:** Rt = 2.38 min (mixture of stereoisomers), **21b:** Rt = 2.88 min (mixture of stereoisomers). |
| | 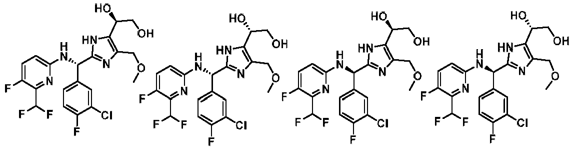 | |
| **22a, 22b** | 1-(2-(bis(4-chloro-2-methoxyphenyl)methyl)-5-(methoxymethyl)-1H-imidazol-4-yl)ethane-1,2-diol | LCMS m/z = 467 [M+H]+;Chiral SFC: column: CHIRALPAK IC (4.6 × 150 mm), 5 µm, co-solvent: 0.3% iPrNH2 in iPiOH; flow: 3 mL/min; % of co-solvent: 30%; ABPR: 1500 psi; T: 35 °C; **22a:** Rt = 2.61 min (first eluting stereoisomer), |
| | 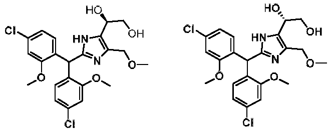 | |
| | | **22b:** Rt = 3.28 min (second eluting stereoisomer). |
| **29** | 2-(2-(bis(3-chloro-4-fluorophenyl)methyl)-4-methyl-1H-imidazol-5-yl)-2-methoxyethan-1-ol | As a mixture of stereoisomers LCMS m/z = 427 [M+H]+; Rt = 2.61 min (method S) |
| | 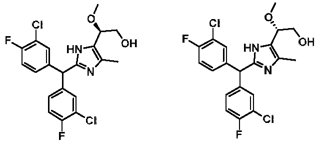 | |
| **30** | 1-(2-(bis(3-chloro-4-fluorophenyl)methyl)-4-methyl-1H-imidazol-5-yl)-2-methoxyethan-1-ol | As a mixture of stereoisomers LCMS m/z = 427 [M+H]+; Rt = 2.61 min (method S) |
| | 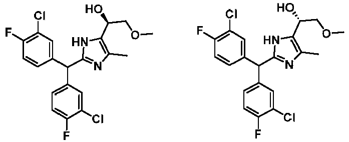 | |

### Example 2

### 3-(2-(bis(3-chloro-4-fluorophenyl)methyl)-5-methyl-1H-imidazol-4-yl)azetidin-3-ol

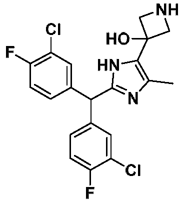

Step 1: To a stirred solution of tert-butyl 3-oxoazetidine-1-carboxylate (0.091 g, 0.53 mmol) in THF (1.0 mL) was added LaCl₃ (0.6M in THF) (0.9 mL, 0.53 mmol) at 0 °C and stirred at ambient temperature for 30 minutes. In another round bottom flask, a stirred solution of 2-[bis(3-chloro-4-fluorophenyl)methyl]-4-bromo-5-methyl-1-{[2-(trimethylsilyl)ethoxy]methyl}-1H-imidazole (intermediate 4, 0.1 g, 0.17 mmol in THF (4.0 mL) was added nBuLi (1.76M in THF) (0.25 mL, 0.45 mmol) at -78 °C and stirred for 2 minutes. The previously prepared solution of tert-butyl 3-oxoazetidine-1-carboxylate in THF was added dropwise at -78 °C and subsequently stirred at ambient temperature for 15 minutes. The reaction mixture was quenched with saturated NH₄Cl solution, diluted with EtOAc and washed with H₂O and brine. The organic extract was dried over anhydrous Na₂SO₄, filtered and concentrated under reduced pressure. The crude product was purified by flash chromatography (SiO₂, 0-30% EtOAc/Hex) to afford tert-butyl 3-{2-[bis(3-chloro-4-fluorophenyl)methyl]-5-methyl-1-{[2-(trimethylsilyl)ethoxy]methyl}-1H-imidazol-4-yl}-3-hydroxyazetidine-1-carboxylate.

Step 2: TFA (3.0 mL) was added to tert-butyl 3-{2-[bis(3-chloro-4-fluorophenyl)methyl]-5-methyl-1-{[2-(trimethylsilyl)ethoxy]methyl}-1H-imidazol-4-yl}-3-hydroxyazetidine-1-carboxylate (0.2 g, 0.31 mmol) at 0 °C and stirred at ambient temperature for 16 h. The mixture was poured into ice cold NaHCO₃ solution and extracted with DCM. Combined organic extracts was washed H₂O and brine, dried over Na₂SO₄, filtered and concentrated to get crude product which was purified by prep-HPLC purification to afford the title compound (0.005 g, 4%).

**Example 2:** LCMS m/z = 424 [M+H]⁺; ¹H-NMR (DMSO-D6, 400 MHz, 100 °C): δ 11.61-11-72 (m, 1H), 7.44-7.53 (m, 1H), 7.27-7.38 (m, 6H), 7.07 (brs, 2H), 6.75 (s, 1H), 4.29 (s, 1H), 3.63-3.65 (m, 1H), 2.08-2.11 (m, 4H).

### Example 3a, 3b

### 1-(2-(bis(3-chloro-4-fluorophenyl)methyl)-4-methyl-1H-imidazol-5-yl)ethan-1-ol

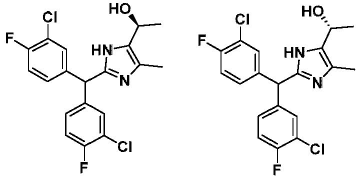

Step 1: A stirred solution of 2-[bis(3-chloro-4-fluorophenyl)methyl]-4-iodo-5-methyl-1-{[2-(trimethylsilyl)ethoxy]methyl}-1H-imidazole (Intermediate 2, 0.6 g, 0.99 mmol) in DMF (4.0 mL) was degassed with N₂ gas for 5 min. Tributyl(1-ethoxyethenyl)stannane (0.66 mL, 1.97 mmol) and Pd(PPh₃)₄ (0.11 g, 0.098 mmol) were added and stirred at 100 °C for 18h. The reaction mixture was cooled and stirred with KF solution for 10 min and then diluted with EtOAc. The organic layer was separated and washed with H₂O and brine and subsequently dried over Na₂SO₄ and concentrated under reduced pressure to obtained crude product which was purified with combi-flash column chromatography (0-30% EtOAc/Hex) to obtain 1-{2-[bis(3-chloro-4-fluorophenyl)methyl]-5-methyl-1-{[2-(trimethylsilyl)ethoxy]methyl}-1H-imidazol-4-yl}ethan-1-one (0.2 g, 39%). LCMS: m/z = 525 [M+H]⁺

Step 2: To a stirred solution of 1-{2-[bis(3-chloro-4-fluorophenyl)methyl]-4-methyl-1-{[2-(trimethylsilyl)ethoxy]methyl}-1H-imidazol-5-yl}ethan-1-one (0.35 g, 0.67 mmol) in MeOH (5 mL) was added NaBH₄ (38 mg, 0.10 mmol) at 0 °C. The reaction mixture was stirred at ambient temperature for 16h. MeOH was removed before H₂O was added and the mixture extracted with EtOAc. Combined organic layers were dried over Na₂SO₄ and concentrated under reduced pressure to obtain 1-{2-[bis(3-chloro-4-fluorophenyl)methyl]-4-methyl-1-{[2-(trimethylsilyl)ethoxy]methyl}-1H-imidazol-5-yl}ethan-1-one (0.53 g) as crude product. LCMS: m/z = 527 [M+H]⁺

Step 3: To a stirred solution of 1-{2-[bis(3-chloro-4-fluorophenyl)methyl]-4-methyl-1-{[2-(trimethylsilyl)ethoxy]methyl}-1H-imidazol-5-yl}ethan-1-ol (230 mg, 0.44 mmol) in DCM (4 mL) was added TFA (0.8 mL). The reaction mixture was stirred at ambient temperature for 16h. The reaction mixture was concentrated and diluted with DCM (30 mL). The organic layer was washed with NaHCO₃ solution and brine, dried over Na₂SO₄ and concentrated to get the crude product, which was purified by reverse-phase prep HPLC followed by the chiral separation using chiral SFC to yield the title compound.

LCMS m/z = 397 [M+H]⁺; ¹H-NMR (400 MHz , 100°C, DMSO-d₆): δ 11.28-11.20 (m, 1H), 7.53 (m 2H), 7.32-7.27 (m, 4H), 5.50-5.47 (m, 1H), 4.78-4.68 (m, 1H), 2.17-2.09 (m, 3H), 1.36-1.34 (m, 3H). Chiral HPLC: column: I-Cellulose Z (21.1 x 250 mm )5 µm; co-solvent: 0.1% NH₂ (MeOH) in ACN/iPrOH (1:1), flow: 3 mL/min; % of co-solvent: 25%; ABPR: 110 bar: 35 °C; **Example 3a:** Yield: 0.013 g, (36%). Rt = 2.33 min (first eluting with unknown absolute configuration). **Example 3b:** Yield: 0.011 g, (31%). ; Rt = 3.01 min (second eluting with unknown absolute configuration).

The following examples may be prepared from the appropriate intermediates and reagents, potentially employing protection strategies, using similar methods to those described for examples 2 and 3. The compounds may be obtained as free base or as a salt e.g. formate or trifluoro acetate. Single stereoisomers may be obtained by chiral separation, either on intermediate or final stage. The absolute configuration remains unknown and cannot be deduced from the order of representations.

| Example | Name/Structure | Analytical Data |
|---|---|---|
| **7** | 3-(2-(bis(3-chloro-4-fluorophenyl)methyl)-5-methyl-1H-imidazol-4-yl)pyrrolidin-3-ol | As mixture of 2 stereoisomers (formate salt) LCMS m/z = 438 [M+H]⁺; Rₜ= 2.37 min (method S) |
| | 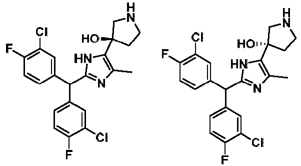 | |
| **9** | 4-(2-(bis(3-chloro-4-fluorophenyl)methyl)-5-methyl-1H-imidazol-4-yl)piperidin-4-ol | LCMS m/z = 454 [M+H]⁺; Rₜ = 2.32 min (method S) |
| | 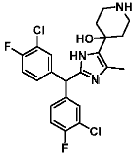 | |
| **10** | 2-(2-(bis(3-chloro-4-fluorophenyl)methyl)-4-methyl-1H-imidazol-5-yl)-1,3-dimethoxypropan-2-ol | LCMS m/z = 471 [M+H]⁺; Rₜ= 2.63 min (method S) |
| | 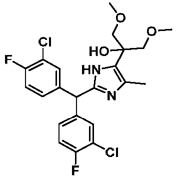 | |
| **12a, 12b** | 2-(2-(bis(3-chloro-4-fluorophenyl)methyl)-5-methyl-1H-imidazol-4-yl)propane-1,2-diol | LCMS m/z = 427 [M+H]⁺; Chiral SFC: C Amylose A (4.6 x 250 mm), 5u, co-solvent: 0.3% iPrNH₂ in MeOH/iPrOH (20:80); flow: 3 mL/min; % of co-solvent: 25%; ABPR: 1450 psi; T: 35 °C; **12a** (first eluting): Rt = 2.66 min, **12b** (second eluting): Rt = 3.12 min. |
| | 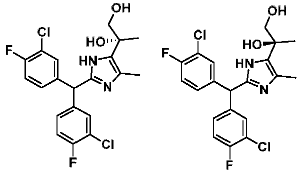 | |
| **13** | 2-(2-(bis(3-chloro-4-fluorophenyl)methyl)-5-methyl-1H-imidazol-4-yl)propan-2-ol | LCMS m/z = 410 [M+H]⁺; Rₜ= 2.61 min (method I) |
| | 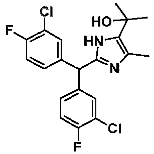 | |
| **15** | 4-(2-(bis(4-chloro-2-methoxyphenyl)methyl)-4-methyl-1H-imidazol-5-yl)piperidin-4-ol | LCMS m/z = 476 [M+H]⁺; Rₜ= 2.21 min (method S) |
| | 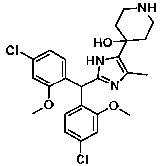 | |
| **18a , 18b** | 3-(2-(bis(4-chloro-2-methoxyphenyl)methyl)-4-methyl-1H-imidazol-5-yl)pyrrolidin-3-ol | LCMS m/z = 462 [M+H]⁺; Chiral SFC: CHIRALCEL OX-H (4.6 × 150 mm), 3.5 µm, co-solvent: 0.3% iPrNH₂ in ACN/iPrOH (1:1); flow: 4 mL/min; % of co-solvent: 40%; ABPR: 1500 psi; T: 35 °C; **18a** (first eluting): Rt = 4.75 min, **18b** (second eluting): Rt = 5.68 min. |
| | 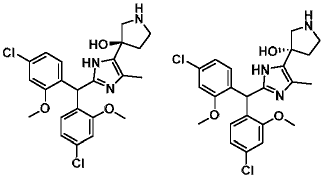 | |
| **20** | 4-(2-(bis(4-chloro-2-methoxyphenyl)methyl)-4-methyl-1H-imidazol-5-yl)-1-methylpiperidin-4-ol | LCMS m/z = 490 [M+H]⁺; Rₜ = 2.46 min (method S) |
| | 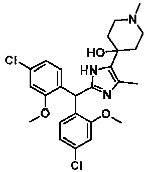 | |
| **23** | 3-amino-1-(2-(bis(4-chloro-2-methoxyphenyl)methyl)-4-methyl-1H-imidazol-5-yl)cyclobutan-1-ol | LCMS m/z = 462 [M+H]⁺; Rₜ= 1.84 min (method S) |
| | 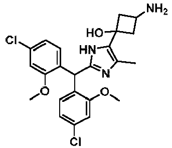 | |

### Example 16a, 16b

### 2-amino-1-(2-(bis(3-chloro-4-fluorophenyl)methyl)-5-methyl-1H-imidazol-4-yl)ethan-1-ol

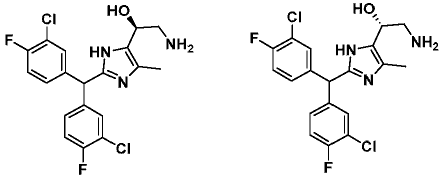

Step 1: To a stirred solution of 1,3-dibromo-5,5-dimethylimidazolidine-2,4-dione (0.45 g, 1.6 mmol) in ACN (6 mL) and water (2 mL) was added 2-[bis(3-chloro-4-fluorophenyl)methyl]-5-ethenyl-4-methyl-1-{[2-(trimethylsilyl)ethoxy]methyl}-1H-imidazole (step 1, example 1) (1.0 g, 1.96 mmol) in ACN (6 mL) at 0°C, The reaction mixture was stirred for 3 h and then quenched by the addition of 15% Na₂S₂O₃ (25 mL) followed by extraction with EtOAc. The combined organic extract was dried over Na₂SO₄, filtered and concentrated under reduced pressure to get a mixture of 2-{2-[bis(3-chloro-4-fluorophenyl)methyl]-4-methyl-1-{[2-(trimethylsilyl)ethoxy]methyl}-1H-imidazol-5-yl}-2-bromoethan-1-ol and 1-{2-[bis(3-chloro-4-fluorophenyl)methyl]-4-methyl-1-{[2-(trimethylsilyl)ethoxy]methyl}-1H-imidazol-5-yl}-2-bromoethan-1-ol. It was forwarded for the next step without any further purification. LCMS: m/z = 606 [M+H]⁺

Step 2: To a stirred solution of the intermediate from step 1 (3.25 g, 5.36 mmol) in DMF (9 mL) and water (1 mL) was added NaN₃ (0.52 g, 8.04 mmol) and reaction mixture was stirred at 80 °C for 16 h. Subsequently the reaction mixture was extracted with EtOAc (150 mL) and washed with water (50 mL) and brine (50 mL). The organic extract was dried over anhydrous Na₂SO₄, filtered and concentrated under reduced pressure. The crude material was purified by flash chromatography (SiO₂, 10-50% EtOAc/Hex) to afford 2-azido-1-{2-[bis(3-chloro-4-fluorophenyl)methyl]-4-methyl-1-{[2-(trimethylsilyl)ethoxy]methyl}-1H-imidazol-5-yl}ethan-1-ol (1^{st} eluting isomer) and its regioisomer (2^{nd} eluting isomer).

Step 3: To a stirred solution of 2-azido-1-(2-(bis(3-chloro-4-fluorophenyl)methyl)-4-methyl-1-((2-(trimethylsilyl)ethoxy)methyl)-1H-imidazol-5-yl)ethan-1-ol 1st eluting regio-isomer) (0.45 g, 0.79 mmol) in THF (12 mL) and water (3 mL) was added PPh₃ (0.31 g, 1.19 mmol) and the reaction mixture stirred at ambient temperature for 16 h. Subsequently the mixture was concentrated under reduced pressure to get the crude product that was directly used for the next step. LCMS: m/z = 542 [M+H]+

Step 4: To a stirred solution of 2-amino-1-(2-(bis(3-chloro-4-fluorophenyl)methyl)-4-methyl-1-((2-(trimethylsilyl)ethoxy)methyl)-1H-imidazol-5-yl)ethan-1-ol (0.5 g, 0.92 mmol) in THF (5 mL) was added Et₃N (0.39 mL, 2.77 mmol) and Boc-anhydride (0.32 mL, 1.38 mmol) and stirred for 16 h before the reaction mixture was concentrated under reduced pressure. The crude product was purified by flash chromatography (SiO₂, 0-100% EtOAc/Hex) to afford tert-butyl (2-(2-(bis(3-chloro-4-fluorophenyl)methyl)-4-methyl-1-((2-(trimethylsilyl)ethoxy)methyl)-1H-imidazol-5-yl)-2-hydroxyethyl)carbamate (0.28 g, 47%). LCMS: m/z = 642 [M+H]⁺

Chiral separation via SFC yields single enantiomers (first eluting 0.10 g, second eluting 0.12 g that were taken to the next step.

Step 5: To a stirred solution of the first eluting peak from step 4 (0.1 g, 0.16 mmol)) in DCM (10 mL) was added TFA (4.0 mL) at 0 °C and stirred at ambient temperature for 16 h. After reaction was completed, the reaction mixture was concentrated under reduced pressure. Crude product was purified by prep-HPLC purification to afford the title compound (example 16a).

Example 16b was obtained in analogy from the second eluting peak.

LCMS m/z = 412 [M+H]⁺; ¹H-NMR (400 MHz, 25°C, DMSO-d₆): δ 7.54 (s, 2H), 7.34-7.36 (m, 4H), 5.49 (s, 1H), 4.40 (brs, 1H), 2.66-2.71 (m, 2H), 2.10 (s, 3H). Chiral SFC: column: Cellulose SC (4.6 × 250 mm) 5 µm; co-solvent: 0.3% iPrNH₂ in MeOH, flow: 3 mL/min; % of co-solvent: 30%; ABPR: 100 bar: 35 °C; **Example 16a** (first eluting with unknown absolute configuration): Yield: 0.022 g, (34%).Rt = 1.84 min. **Example 16b** (second eluting with unknown absolute configuration): Yield: 0.015 g; Rt = 1.85 min to 2.02 min.

### Example 28a, 28b

### 2-amino-2-(2-(bis(3-chloro-4-fluorophenyl)methyl)-5-methyl-1H-imidazol-4-yl)ethan-1-ol

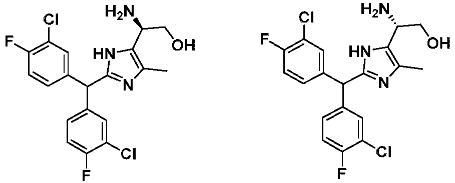

The synthesis was performed in analogy to example 16a, 16b from the 2^{nd} eluting isomer of step 2 employing step 3 followed by chiral separation and step 5 for the single stereoisomers.

**Example 28a** (first eluting with unknown absolute configuration), **28b** (second eluting with unknown absolute configuration): LCMS m/z = 412 [M+H]⁺; ¹H-NMR (400 MHz , 100°C, DMSO-d₆): δ 11.30 (bs, 1H), 7.51 (d, *J=* 6.8 Hz, 2H), 7.31-7.27 (m, 4H), 5.50 (s, 1H), 4.38 (bs, 1H), 3.87 (bs, 1H), 3.48 (bs, 2H), 2.13 (s, 3H).

### Example 25

### N-((4-(3-aminooxetan-3-yl)-1H-imidazol-2-yl)(3-chloro-4-fluorophenyl)methyl)-5-fluoro-6-methylpyridin-2-amine

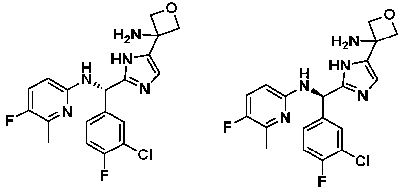

Step 1: To a stirred solution of 4-bromo-1H-imidazole (10.0 g, 68.0 mmol) in DMF (50 mL) was added NaH [60% in Mineral Oil] (4.1 g, 102 mmol) at 0 °C and stirred for 5 minutes. Then SEM-Cl (13.3 mL, 74.8 mmol) was added dropwise at 0 °C. The reaction mixture was stirred at ambient temperature for 2 h. Then the reaction mixture was quenched with saturated NH₄Cl solution, diluted with EtOAc (250 mL) and washed with H₂O (100 mL) and brine (3 × 100 mL). The organic extract was dried over anhydrous Na₂SO₄, filtered and concentrated under reduced pressure. The crude product was purified by flash chromatography (SiO₂, 0-50% EtOAc/Hex) to afford 4-bromo-1-{[2-(trimethylsilyl)ethoxy]methyl}-1H-imidazole (15.0 g, 79%).

Step 2: To a solution of diisopropylamine (6.5 mL, 46 mmol) in THF (100 mL) was added n-BuLi (1.8M in Hexane; 17.2 mL, 31 mmol) dropwise at 0 °C. After 30 minutes stirring at 0 °C, a solution of 4-bromo-1-{[2-(trimethylsilyl)ethoxy]methyl}-1H-imidazole (7.2 g, 26 mmol) in THF (10 mL) was added dropwise at -78 °C and the reaction mixture stirred for 1 h. A solution tert-butyl(chloro)dimethylsilane (4.6 g, 31 mmol) in THF (5.0 mL) was added dropwise at -78 °C. The resulting reaction mixture was stirred at ambient temperature for 2 h before it was quenched with saturated NH₄Cl solution and extracted with EtOAc (2 × 10 mL). The combined organic layer was washed with brine (10 mL), dried over anhydrous Na₂SO₄, filtered and concentrated under reduced pressure. The resulting crude material was purified by combi-flash column chromatography (SiO₂, 0-30% EtOAc/Hex) to yield 5-bromo-2-(tert-butyldimethylsilyl)-1-((2-(trimethylsilyl)ethoxy)methyl)-1H-imidazole (10.0 g, 98%).

Step 3: To a solution of 5-bromo-2-(tert-butyldimethylsilyl)-1-((2-(trimethylsilyl)ethoxy)methyl)-1H-imidazole (4.0 g, 10.1 mmol) in THF (50 mL) was added n-BuLi (1.8M in Hexane) (6.8 mL, 12.2 mmol) dropwise at -78 °C and stirred for 5 minutes. A solution of 2-methyl-N-(oxetan-3-ylidene)propane-2-sulfinamide (2.68 g, 15.29 mmol) in THF (10.0 mL) was added dropwise to reaction mixture at -78 °C. The reaction mixture was stirred for 3 h, quenched with saturated NH₄Cl solution, diluted with EtOAc (150 mL) and washed with H₂O (50 mL) and brine (50 mL). The organic extract was dried over anhydrous Na₂SO₄, filtered and concentrated under reduced pressure. The crude product was purified by flash chromatography (SiO₂, 0-20% EtOAc/Hex) to afford N-(3-(2-(tert-butyldimethylsilyl)-1-((2-(trimethylsilyl)ethoxy)methyl)-1H-imidazol-5-yl)oxetan-3-yl)-2-methylpropane-2-sulfinamide (2.0 g, 40%). LCMS: m/z = 488 [M+H]⁺

Step 4: To a solution of N-(3-(2-(tert-butyldimethylsilyl)-1-((2-(trimethylsilyl)ethoxy)methyl)-1H-imidazol-5-yl)oxetan-3-yl)-2-methylpropane-2-sulfinamide (1.3 g, 2.66 mmol) in THF (15 mL) was added TBAF (1M in THF) (5.3 mL, 5.31 mmol). The reaction was stirred at ambient temperature for 1 h, then diluted with EtOAc (100 mL) and washed with H₂O (50 mL) and brine (50 mL). Organic extract was dried over anhydrous Na₂SO₄, filtered and concentrated under reduced pressure. Crude product was purified by flash chromatography (SiO₂, 0-40% EtOAc-EtOH (3:1)/Hex) to afford 2-methyl-N-(3-(1-((2-(trimethylsilyl)ethoxy)methyl)-1H-imidazol-5-yl)oxetan-3-yl)propane-2-sulfinamide (0.6 g, 60%). LCMS: m/z = 374 [M+H]⁺

Step 5: To a solution of N-(3-(2-(tert-butyldimethylsilyl)-1-((2-(trimethylsilyl)ethoxy)methyl)-1H-imidazol-5-yl)oxetan-3-yl)-2-methylpropane-2-sulfinamide (0.35 g, 0.93 mmol) in THF (2.5 mL) was added nBuLi (1.7 M) (1.4 mL , 2.34 mmol) at -78 °C and stirred at this temperature for 15 min then at 0 °C for 30 min. A solution of N-[(3-chloro-4-fluorophenyl)methylidene]-5-fluoro-6-methylpyridin-2-amine (0.50 g, 1.87 mmol; prepared from 3-chloro-4-fluorobenzaldehyde and 5-fluoro-6-methylpyridin-2-amine in toluene) in THF (0.5 mL) was added to the reaction mixture at 0 °C and stirred at ambient temperature for 16 h. The reaction mixture was quenched with saturated NH₄Cl solution, diluted with EtOAc (150 mL) and washed with H₂O (50 mL) and brine (50 mL). The organic extract was dried over anhydrous Na₂SO₄, filtered and concentrated under reduced pressure. The crude product was purified by flash chromatography (SiO₂, 0-70% EtOAc/Hex) to afford N-(3-(2-((3-chloro-4-fluorophenyl)((5-fluoro-6-methylpyridin-2-yl)amino)methyl)-1-((2-(trimethylsilyl)ethoxy)methyl)-1H-imidazol-5-yl)oxetan-3-yl)-2-methylpropane-2-sulfinamide (0.32 g, 53.41%). LCMS: m/z = 640 [M+H]⁺

Step 6: To a stirred solution of N-(3-(2-((3-chloro-4-fluorophenyl)((5-fluoro-6-methylpyridin-2-yl)amino)methyl)-1-((2-(trimethylsilyl)ethoxy)methyl)-1H-imidazol-5-yl)oxetan-3-yl)-2-methylpropane-2-sulfinamide (0.3 g, 0.47 mmol) in DCM (2.0 mL) was added TFA (3.0 mL) at 0 °C. The reaction mixture was stirred ambient temperature for 16 h. Then the reaction mixture was concentrated under reduced pressure. The crude product was purified by prep-HPLC purification to afford the title compound (0.06 g, 31%).

**Example 25** (as mixture of stereoisomers): LCMS m/z = 406 [M+H]⁺; ¹H-NMR (400 MHz , 100°C, DMSO-d₆): δ 11.60 (brs, 1H), 7.61 (m, 1H), 7.41 (m, 1H), 7.21-7.30 (m, 2H), 6.89-6.98 (m, 2H), 6.52 (m, 1H), 6.23 (d, 1H) 4.65-4.76 (m, 2H), 4.46-4.52 (m, 2H), 2.32 (m, 2H), 2.24 (s, 3H).

The following examples may be prepared from the appropriate intermediates and reagents, potentially employing protection strategies, using similar methods to those described for examples 16, 25 and 28. The compounds may be obtained as free base or as a salt e.g. formate or trifluoro acetate. Single stereoisomers may be obtained by chiral separation, either on intermediate or final stage. The absolute configuration remains unknown and cannot be deduced from the order of representations.

| Ex. | Name/Structure | Analytical Data |
|---|---|---|
| **11** | 2-amino-1-(2-((3-chloro-4-fluorophenyl)((5-fluoro-6-methylpyridin-2-yl)amino)methyl)-5-methyl-1H-imidazol-4-yl)ethan-1-ol | As mixture of stereoisomers (formate salt) LCMS m/z = 408 |
| | 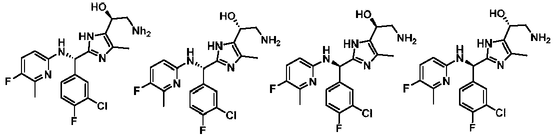 | [M+H]⁺; Rt= 2.14 min (method S) |
| **24** | 1-(2-(bis(3-chloro-4-fluorophenyl)methyl)-4-methyl-1H-imidazol-5-yl)ethan-1-amine | As mixture of stereoisomers (TFA salt) LCMS m/z = 398 [M+H]⁺; Rₜ = 2.11 min (method S) |
| | 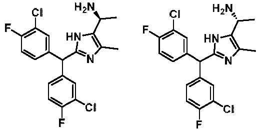 | |
| **26** | 1-(2-((3-chloro-4-fluorophenyl)((4,4-difluorocyclohexyl)oxy)methyl)-4-methyl-1H-imidazol-5-yl)ethan-1-amine | As mixture of stereoisomers (formate salt) LCMS m/z = 402[M+H]⁺; Rₜ = 4.05, 4.19 min (method V) |
| | 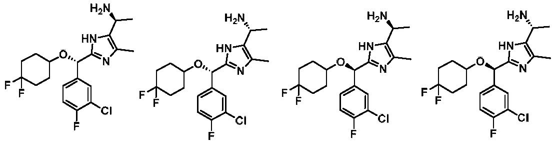 | |
| **27** | *N*-((4-(4-aminotetrahydro-2H-pyran-4-yl)-1H-imidazol-2-yl)(3-chloro-4-fluorophenyl)methyl)-5-fluoro-6-methylpyridin-2-amine | As mixture of stereoisomers LCMS m/z = 434 [M+H]⁺; Rₜ = 2.51 min (method S) |
| | 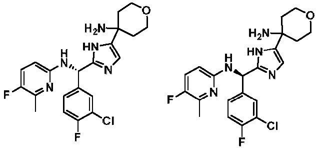 | |

The following prophetic examples could be synthesized according to the general schemes and by analogy to the synthetic procedures described above:
17 1-(2-((3-chloro-4-fluorophenyl)((5-fluoro-6-methylpyridin-2-yl)amino)methyl)-1H-imidazol-4-yl)ethane-1,2-diol
31 2-(azetidin-1-yl)-2-(2-(bis(3-chloro-4-fluorophenyl)methyl)-5-methyl-1H-imidazol-4-yl)ethan-1-ol
32 1-(2-(bis(4,5-difluoro-2-methoxyphenyl)methyl)-5-methyl-1H-imidazol-4-yl)ethane-1,2-diol
33 2-amino-2-(2-((3-chloro-4-fluorophenyl)((5-fluoro-6-methylpyridin-2-yl)amino)methyl)-4-methyl-1H-imidazol-5-yl)ethan-1-ol
34 2-amino-1-(2-((3-chloro-4-fluorophenyl)((5-fluoro-6-methylpyridin-2-yl)amino)methyl)-4-methyl-1H-imidazol-5-yl)ethan-1-ol
35 azetidin-3-yl(2-(bis(3-chloro-4-fluorophenyl)methyl)-4-methyl-1H-imidazol-5-yl)methanol
36 5-(1-(azetidin-1-yl)-2-methoxyethyl)-2-(bis(3-chloro-4-fluorophenyl)methyl)-4-methyl-1H-imidazole
37 2-(azetidin-1-yl)-1-(2-(bis(3-chloro-4-fluorophenyl)methyl)-4-methyl-1H-imidazol-5-yl)ethan-1-ol
38 N-((5-(1-aminoethyl)-4-methyl-1H-imidazol-2-yl)(3-chloro-4-fluorophenyl)methyl)-5-fluoro-6-methylpyridin-2-amine
39 N-((5-(2-amino-1,3-dimethoxypropan-2-yl)-1H-imidazol-2-yl)(3-chloro-4-fluorophenyl)methyl)-5-fluoro-6-methylpyridin-2-amine
40 N-((5-((azetidin-3-yloxy)methyl)-4-methyl-1H-imidazol-2-yl)(3-chloro-4-fluorophenyl)methyl)-5-fluoro-6-methylpyridin-2-amine
41 4-(2-((3-chloro-4-fluorophenyl)((4-(trifluoromethyl)cyclohexyl)oxy)methyl)-5-methyl-1H-imidazol-4-yl)piperidin-4-ol
42 4-(2-((3-chloro-4-fluorophenyl)((4-(trifluoromethyl)cyclohexyl)oxy)methyl)-5-(methoxymethyl)-1H-imidazol-4-yl)piperidin-4-ol
43 4-(2-((3-chloro-4-fluorophenyl)((4-(trifluoromethyl)cyclohexyl)oxy)methyl)-5-(methoxymethyl)-1H-imidazol-4-yl)-1-methylpiperidin-4-ol
44 4-(2-((3-chloro-4-fluorophenyl)((4-(trifluoromethyl)cyclohexyl)oxy)methyl)-5-methyl-1H-imidazol-4-yl)-1-methylpiperidin-4-ol
45 2-(2-(bis(3-chloro-4-fluorophenyl)methyl)-5-methyl-1H-imidazol-4-yl)-2-(dimethylamino)ethan-1-ol
46 2-amino-2-(2-((3-chloro-4-fluorophenyl)((5-fluoro-6-methylpyridin-2-yl)amino)methyl)-5-methyl-1H-imidazol-4-yl)ethan-1-ol
47 N-((4-(1-amino-2-methoxyethyl)-5-methyl-1H-imidazol-2-yl)(3-chloro-4-fluorophenyl)methyl)-5-fluoro-6-methylpyridin-2-amine
48 2-(2-((3-chloro-4-fluorophenyl)((5-fluoro-6-methylpyridin-2-yl)amino)methyl)-5-methyl-1H-imidazol-4-yl)-2-(methylamino)ethan-1-ol
49 2-(2-((3-chloro-4-fluorophenyl)((5-fluoro-6-methylpyridin-2-yl)amino)methyl)-5-methyl-1H-imidazol-4-yl)-2-(dimethylamino)ethan-1-ol
50 N-((3-chloro-4-fluorophenyl)(4-(1-(dimethylamino)-2-methoxyethyl)-5-methyl-1H-imidazol-2-yl)methyl)-5-fluoro-6-methylpyridin-2-amine
51 N-((4-(1-amino-2,2-difluoroethyl)-5-methyl-1H-imidazol-2-yl)(3-chloro-4-fluorophenyl)methyl)-5-fluoro-6-methylpyridin-2-amine
52 N-((4-(1-amino-2-(difluoromethoxy)ethyl)-5-methyl-1H-imidazol-2-yl)(3-chloro-4-fluorophenyl)methyl)-5-fluoro-6-methylpyridin-2-amine
53 N-((4-(1-amino-2-(trifluoromethoxy)ethyl)-5-methyl-1H-imidazol-2-yl)(3-chloro-4-fluorophenyl)methyl)-5-fluoro-6-methylpyridin-2-amine
54 2-amino-2-(2-((3-chloro-4-fluorophenyl)((4-(trifluoromethyl)cyclohexyl)oxy)methyl)-5-methyl-1H-imidazol-4-yl)ethan-1-ol
55 1-(2-((3-chloro-4-fluorophenyl)((4-(trifluoromethyl)cyclohexyl)oxy)methyl)-5-methyl-1H-imidazol-4-yl)-2-methoxyethan-1-amine
56 2-(2-((3-chloro-4-fluorophenyl)((4-(trifluoromethyl)cyclohexyl)oxy)methyl)-5-methyl-1H-imidazol-4-yl)-2-(dimethylamino)ethan-1-ol
57 1-(2-((3-chloro-4-fluorophenyl)((4-(trifluoromethyl)cyclohexyl)oxy)methyl)-5-methyl-1H-imidazol-4-yl)-2-(difluoromethoxy)ethan-1-amine
58 1-(2-((3-chloro-4-fluorophenyl)((4-(trifluoromethyl)cyclohexyl)oxy)methyl)-5-methyl-1H-imidazol-4-yl)-2-(trifluoromethoxy)ethan-1-amine
59 N-((3-chloro-4-fluorophenyl)(4-(2-methoxy-1-(methylamino)ethyl)-5-methyl-1H-imidazol-2-yl)methyl)-5-fluoro-6-methylpyridin-2-amine
60 2-amino-2-(2-(bis(4-chloro-2-methoxyphenyl)methyl)-5-methyl-1H-imidazol-4-yl)ethan-1-ol
61 1-(2-(bis(4-chloro-2-methoxyphenyl)methyl)-5-methyl-1H-imidazol-4-yl)-2-methoxyethan-1-amine
62 2-(2-(bis(4-chloro-2-methoxyphenyl)methyl)-5-methyl-1H-imidazol-4-yl)-2-(methylamino)ethan-1-ol
63 2-(2-(bis(4-chloro-2-methoxyphenyl)methyl)-5-methyl-1H-imidazol-4-yl)-2-(dimethylamino)ethan-1-ol
64 1-(2-(bis(4-chloro-2-methoxyphenyl)methyl)-5-methyl-1H-imidazol-4-yl)-2-(difluoromethoxy)ethan-1-amine
65 N-((3-chloro-4-fluorophenyl)(4-(2-(difluoromethoxy)-1-(methylamino)ethyl)-5-methyl-1H-imidazol-2-yl)methyl)-5-fluoro-6-methylpyridin-2-amine
66 2-amino-2-(2-(bis(3-chloro-4-fluorophenyl)methyl)-5-(difluoromethyl)-1H-imidazol-4-yl)ethan-1-ol
67 2-amino-2-(2-(bis(3-chloro-4-fluorophenyl)methyl)-5-(trifluoromethyl)-1H-imidazol-4-yl)ethan-1-ol
68 2-amino-2-(2-(bis(3-chloro-4-fluorophenyl)methyl)-5-chloro-1H-imidazol-4-yl)ethan-1-ol
69 2-amino-2-(2-((3-chloro-4-fluorophenyl)((5-fluoro-6-methylpyridin-2-yl)amino)methyl)-5-methyl-1H-imidazol-4-yl)propan-1-ol
70 4-(1-amino-2-hydroxyethyl)-2-(bis(3-chloro-4-fluorophenyl)methyl)-1H-imidazole-5-carbonitrile

### Electrophysiology: Voltage-Clamp recordings

The following Na_{V}1.8 recombinant cell line was used for recordings: HEK-Na_{V}1.8 (NM_006514.1) with b3 (NM_018400.3).

Sodium currents were measured using the patch clamp technique in the whole-cell configuration using the Qube384 (Sophion A/S, Copenhagen, Denmark) automated voltage clamp platform. "Multi hole" plates were used for the cell line expressing Na_{V}1.8. Appropriate filters (for minimum seal resistance and minimum current size) and series resistance compensation (for high quality sodium channel recordings) were applied. Data was collected at ambient room temperature or 19 °C.

The recording extracellular solution contained (in mM): NaCl 145 mM, KCl 4 mM, CaCl₂ 2 mM, MgCl₂ 1 mM, HEPES 10 mM, Glucose 10 mM, pH 7.4 (NaOH). The intracellular recording solution contained (in mM): CsF 120 mM, CsCl 20 mM, NaCl 10 mM, EGTA 10 mM, HEPES 10 mM, pH 7.2 (CsOH). Currents were recorded at 25 kHz sampling frequency and filtered at 5 kHz. Series resistance compensation was applied at 65%.

Vehicle (VEH) is the control condition where cells are exposed to 0.3 % DMSO without compound. All runs include VEH controls being exposed to the same voltage protocols to assess non-compound related phenomena such as run down and then used to isolate compound dependent effects on currents.

To check for *state-dependent inhibition*, the following voltage-sequence was applied every 20 seconds:
From a resting membrane potential of -120 mV, the first test pulse (P1; 20 ms to -10 mV) was applied to check for channels in the Resting State followed by a brief recovery (100 ms to -120 mV), then holding the membrane voltage to V_{1/2}(4 seconds at voltage to obtain half the channel at Rest and half Inactivated) with a subsequent second test pulse (P2; 20 ms to -10 mV) to check for channels in the Inactivated State, followed by another brief recovery (20 ms to -120 mV) and a final third test pulse (P3; 20 ms to -10 mV) to check for recovered channels.

To check for *frequency-dependent inhibition*, a 10 Hz protocol and a 20 Hz protocol were applied; namely,
From a resting membrane potential of -120 mV, 40-pulses (10 ms to -10 mV) was applied at 10 Hz (at 100 ms between pulses) and then at 20 Hz (at 50 ms between pulses).

Each parameter was recorded (P1, P2, P3, P40 from 10Hz and P40 from 20Hz) during a control period (lasting ~ 5 minutes) when establishing the baseline and during compound period (lasting ~ 12 minutes) when test compound (or vehicle) was applied. For each parameter, the value at the end of the compound period was normalized to the vehicle baseline; as follows $Normalized Inhibition \left({Norm}_{CPD}\right) = \frac{CPD {Value}_{end CPD period}}{VEH {Value}_{end Control Period}}$

To adjust for any variance in the Na⁺ current signal during the compound period (owing to cumulative inactivation independent of compound or shifts in biophysics over time), a dedicated segment of the recording wells in each 384 plate were dedicated to having only vehicle exposure. These vehicle-only recordings were used to correct for any apparent "run-up" or "run-down" in the experiment. $Normalized Inhibition \left({Norm}_{VEH}\right) = \frac{VEH {Value}_{end CPD period}}{VEH {Value}_{end Control Period}}$

The adjusted inhibition was calculated as follows: $% {Inhibiton}_{corrected} = 100 \times \frac{{Norm}_{CPD} - {Norm}_{VEH}}{100 - {Norm}_{VEH}}$

Percent inhibition was determined and IC₅₀ values were calculated using a 4 parameter logistic model within XLFit Software (IDBS, Boston MA): $% {Inhibition}_{corrected} = A + \frac{\left(B-A\right)}{\left(1\right. + {\left(\frac{x}{C}\right)}^{D}}$ where A and B are the maximal and minimum inhibition respectively, C is the IC₅₀ concentration and D is the (Hill) slope.

The potency data of the example compounds are summarized in the table below (category **A:** human NaV1.8 IC₅₀ ≤ 0.1 µM; category **B**: 0.1 µM < human NaV1.8 IC₅₀ ≤ 1 µM; category **C:** 1 µM < human NaV1.8 IC₅₀ ≤ 10 µM; "n.d.": not determined):

| Example | Potency category | | Example | Potency category | | Example | Potency category |
|---|---|---|---|---|---|---|---|
| 1a | A | | 8c | B | | 19b | B |
| 1b | A | | 9 | A | | 20 | A |
| 2 | B | | 10 | A | | 21a | A |
| 3a | A | | 11 | A | | 21b | B |
| 3b | A | | 12a | A | | 22a | A |
| 4a | C | | 12b | A | | 22b | A |
| 4b | B | | 13 | A | | 23 | A |
| 5a | B | | 14a | A | | 24 | A |
| 5b | B | | 14b | A | | 25 | A |
| 6a | n.d. | | 15 | A | | 26 | A |
| 6b | n.d. | | 16a | A | | 27 | A |
| 6c | A | | 16b | A | | 28a | A |
| 7 | A | | 18a | A | | 28b | A |
| 8a | A | | 18b | A | | 29 | A |
| 8b | A | | 19a | A | | 30 | A |

## Claims

1. A compound according to general formula (I) 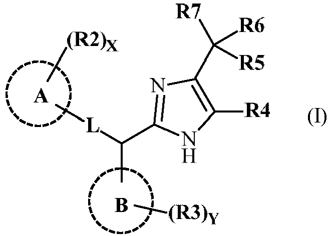 wherein
**A** and **B** independently from one another represent phenyl, 5 to 10-membered heteroaryl, C₃₋₇-cycloalkyl, or 4- to 10-membered heterocycloalkyl;
**R2** and **R3** independently from one another represent F, Cl, CN, C₁₋₄-alkyl, C₃₋₇-cycloalkyl, OH, OC₁₋₄-alkyl, NH₂, NHC₁₋₄-alkyl or N(C₁₋₄-alkyl)₂;
**X** and **Y** independently from one another represent 0, 1, 2, 3 or 4;
**R4** represents H, Cl, CN, C₁₋₄-alkyl or C₃₋₇-cycloalkyl;
**L** represents bond, O, NH or C₁₋₃-alkylene;
**R5** represents H or C₁₋₄-alkyl;
**R6** represents H, C₁₋₄-alkyl, 4- to 7-membered heterocycloalkyl, or C₁₋₃-alkylene-(4- to 7-membered heterocycloalkyl); or
**R5** and **R6** together with the carbon atom to which they are attached form C₃₋₇-cycloalkyl or 4- to 7-membered heterocycloalkyl; and
**R7** represents OH, OC₁₋₄-alkyl, O-4- to 7-membered heterocycloalkyl, NH₂, NHC₁₋₄-alkyl, N(C₁₋₄-alkyl)₂, or N**RaRb** wherein **Ra** and **Rb** together with the nitrogen atom to which they are attached form 4- to 7-membered heterocycloalkyl;
wherein C₁₋₄-alkyl and C₁₋₃-alkylene in each case independently from one another is linear or branched, saturated or unsaturated;
wherein C₁₋₄-alkyl, C₁₋₃-alkylene, C₃₋₇cycloalkyl, 4- to 7-membered heterocycloalkyl and 4- to 10-membered heterocycloalkyl in each case independently from one another are unsubstituted or mono- or polysubstituted with one or more substituents selected from F, C₁₋₄-alkyl, C₁₋₃-alkylene-NH₂, CH₂F, CHF₂, CF₃, OH, OC₁₋₄-alkyl, OCH₂F, OCHF₂, OCF₃, NH₂, NHC₁₋₄-alkyl and N(C₁₋₄-alkyl)₂;
wherein phenyl and 5 to 10-membered heteroaryl in each case independently from one another are unsubstituted or mono- or polysubstituted with one or more substituents selected from F, Cl, CN, C₁₋₄-alkyl, CH₂F, CHF₂, CF₃, OH, OCH₂F, OCHF₂, OCF₃, OC₁₋₄-alkyl, NH₂, NHC₁₋₄-alkyl and N(C₁₋₄-alkyl)₂;
in the form of the free compound or a physiologically acceptable salt thereof.

2. The compound according to claim 1, wherein **R2** represents F, Cl, CH₃, CH₂F, CHF₂, CF₃ or OCH₃.

3. The compound according to claim 1 or 2, wherein **R3** represents F, Cl, CH₃, CH₂F, CHF₂, CF₃ or OCH₃.

4. The compound according to any of the preceding claims, wherein **R4** represents H, Cl, CN, CH₃, CH₂F, CHF₂, CF₃ or CH₂OCH₃.

5. The compound according to any of the preceding claims, wherein **R5** represents H, CH₃ or CH₂OCH₃.

6. The compound according to any of the preceding claims, wherein **R6** represents H, CH₃, CH₂F, CHF₂, CF₃, CH₂OH, CH₂OCH₃, CH₂OCH₂F, CH₂OCHF₂, CH₂OCF₃, CH₂NH₂, azetidine unsubstituted or CH₂-azetidine unsubstituted.

7. The compound according to any of the preceding claims, wherein **R5** and **R6** together with the carbon atom to which they are attached form
cycloalkyl selected from cyclobutyl; in each case unsubstituted or monosubstituted with NH₂; or
heterocycloalkyl selected from oxetane, azetidine, oxolane, pyrrolidine, tetrahydropyran, and piperidine; in each case unsubstituted or monosubstituted with CH₃.

8. The compound according to any of the preceding claims, wherein **R7** represents OH, OCH₃, O-azetidine unsubstituted, NH₂, NHCH₃, N(CH₃)₂, or N**RaRb** wherein **Ra** and **Rb** together with the nitrogen atom to which they are attached form azetidine unsubstituted.

9. The compound according to any of the preceding claims, wherein **L** represents bond, O or N(H).

10. The compound according to any of the preceding claims, wherein **A** represents phenyl, pyridyl or cyclohexyl.

11. The compound according to any of the preceding claims, wherein **B** represents phenyl.

12. The compound according to any of the preceding claims, which is according to general formula (II) or (III) 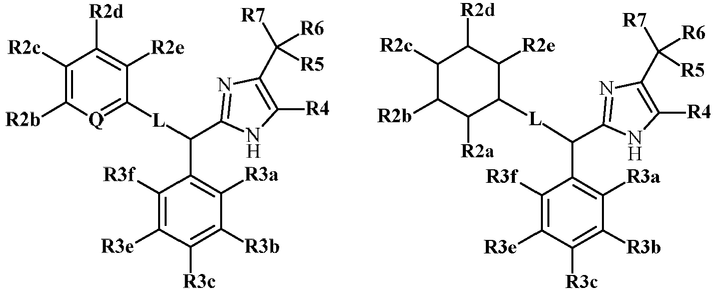 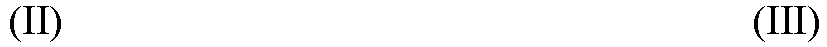 wherein
**L, R4, R5, R6** and **R7** are defined as in any of the preceding claims;
**Q** represents N or C**R2a**;
**R2a, R2b, R2c, R2d** and **R2e** independently from one another represent H, F, Cl, CH₃, CH₂F, CHF₂, CF₃ or OCH₃; with the proviso that not more than 4 of **R2a, R2b, R2c, R2d,** and **R2e** are other than H; **R3a, R3b, R3c, R3d** and **R3e** independently from one another represent H, F, Cl, CH₃, CH₂F, CHF₂, CF₃ or OCH₃; with the proviso that not more than 4 of **R3a, R3b, R3c, R3d** and **R3e** are other than H.

13. The compound according to any of the preceding claims, which is selected from the group consisting of
1 1-(2-(bis(3-chloro-4-fluorophenyl)methyl)-5-methyl-1H-imidazol-4-yl)ethane-1,2-diol
2 3-(2-(bis(3-chloro-4-fluorophenyl)methyl)-5-methyl-1H-imidazol-4-yl)azetidin-3-ol
3 1-(2-(bis(3-chloro-4-fluorophenyl)methyl)-4-methyl-1H-imidazol-5-yl)ethan-1-ol
4 1-(2-((3,4-difluorophenyl)((5-fluoro-6-methylpyridin-2-yl)amino)methyl)-5-methyl-1H-imidazol-4-yl)ethane-1,2-diol
5 1-(2-((3-chloro-4-fluorophenyl)((4,4-difluorocyclohexyl)oxy)methyl)-5-methyl-1H-imidazol-4-yl)ethane-1,2-diol
6 1-(2-((3-chloro-4-fluorophenyl)((5-fluoro-6-methylpyridin-2-yl)amino)methyl)-5-(methoxymethyl)-1H-imidazol-4-yl)ethane-1,2-diol
7 3-(2-(bis(3-chloro-4-fluorophenyl)methyl)-5-methyl-1H-imidazol-4-yl)pyrrolidin-3-ol
8 1-(2-((3-chloro-4-fluorophenyl)((5-fluoro-6-methylpyridin-2-yl)amino)methyl)-5-methyl-1H-imidazol-4-yl)ethane-1,2-diol
9 4-(2-(bis(3-chloro-4-fluorophenyl)methyl)-5-methyl-1H-imidazol-4-yl)piperidin-4-ol
10 2-(2-(bis(3-chloro-4-fluorophenyl)methyl)-4-methyl-1H-imidazol-5-yl)-1,3-dimethoxypropan-2-ol
11 2-amino-1-(2-((3-chloro-4-fluorophenyl)((5-fluoro-6-methylpyridin-2-yl)amino)methyl)-5-methyl-1H-imidazol-4-yl)ethan-1-ol
12 2-(2-(bis(3-chloro-4-fluorophenyl)methyl)-5-methyl-1H-imidazol-4-yl)propane-1,2-diol
13 2-(2-(bis(3-chloro-4-fluorophenyl)methyl)-5-methyl-1H-imidazol-4-yl)propan-2-ol
14 1-(2-(bis(4-chloro-2-methoxyphenyl)methyl)-5-methyl-1H-imidazol-4-yl)ethane-1,2-diol
15 4-(2-(bis(4-chloro-2-methoxyphenyl)methyl)-4-methyl-1H-imidazol-5-yl)piperidin-4-ol
16 2-amino-1-(2-(bis(3-chloro-4-fluorophenyl)methyl)-5-methyl-1H-imidazol-4-yl)ethan-1-ol
17 1-(2-((3-chloro-4-fluorophenyl)((5-fluoro-6-methylpyridin-2-yl)amino)methyl)-1H-imidazol-4-yl)ethane-1,2-diol
18 3-(2-(bis(4-chloro-2-methoxyphenyl)methyl)-4-methyl-1H-imidazol-5-yl)pyrrolidin-3-ol
19 1-(2-((3-chloro-4-fluorophenyl)((6-(difluoromethyl)-5-fluoropyridin-2-yl)amino)methyl)-5-methyl-1H-imidazol-4-yl)ethane-1,2-diol
20 4-(2-(bis(4-chloro-2-methoxyphenyl)methyl)-4-methyl-1H-imidazol-5-yl)-1-methylpiperidin-4-ol
21 1-(2-((3-chloro-4-fluorophenyl)((6-(difluoromethyl)-5-fluoropyridin-2-yl)amino)methyl)-5-(methoxymethyl)-1H-imidazol-4-yl)ethane-1,2-diol
22 1-(2-(bis(4-chloro-2-methoxyphenyl)methyl)-5-(methoxymethyl)-1H-imidazol-4-yl)ethane-1,2-diol
23 3-amino-1-(2-(bis(4-chloro-2-methoxyphenyl)methyl)-4-methyl-1H-imidazol-5-yl)cyclobutan-1-ol
24 1-(2-(bis(3-chloro-4-fluorophenyl)methyl)-4-methyl-1H-imidazol-5-yl)ethan-1-amine
25 N-((4-(3-aminooxetan-3-yl)-1H-imidazol-2-yl)(3-chloro-4-fluorophenyl)methyl)-5-fluoro-6-methylpyridin-2-amine
26 1-(2-((3-chloro-4-fluorophenyl)((4,4-difluorocyclohexyl)oxy)methyl)-4-methyl-1H-imidazol-5-yl)ethan-1-amine
27 N-((4-(4-aminotetrahydro-2H-pyran-4-yl)-1H-imidazol-2-yl)(3-chloro-4-fluorophenyl)methyl)-5-fluoro-6-methylpyridin-2-amine
28 2-amino-2-(2-(bis(3-chloro-4-fluorophenyl)methyl)-5-methyl-1H-imidazol-4-yl)ethan-1-ol
29 2-(2-(bis(3-chloro-4-fluorophenyl)methyl)-4-methyl-1H-imidazol-5-yl)-2-methoxyethan-1-ol
30 1-(2-(bis(3-chloro-4-fluorophenyl)methyl)-4-methyl-1H-imidazol-5-yl)-2-methoxyethan-1-ol
31 2-(azetidin-1-yl)-2-(2-(bis(3-chloro-4-fluorophenyl)methyl)-5-methyl-1H-imidazol-4-yl)ethan-1-ol
32 1-(2-(bis(4,5-difluoro-2-methoxyphenyl)methyl)-5-methyl-1H-imidazol-4-yl)ethane-1,2-diol
33 2-amino-2-(2-((3-chloro-4-fluorophenyl)((5-fluoro-6-methylpyridin-2-yl)amino)methyl)-4-methyl-1H-imidazol-5-yl)ethan-1-ol
34 2-amino-1-(2-((3-chloro-4-fluorophenyl)((5-fluoro-6-methylpyridin-2-yl)amino)methyl)-4-methyl-1H-imidazol-5-yl)ethan-1-ol
35 azetidin-3-yl(2-(bis(3-chloro-4-fluorophenyl)methyl)-4-methyl-1H-imidazol-5-yl)methanol
36 5-(1-(azetidin-1-yl)-2-methoxyethyl)-2-(bis(3-chloro-4-fluorophenyl)methyl)-4-methyl-1H-imidazole
37 2-(azetidin-1-yl)-1-(2-(bis(3-chloro-4-fluorophenyl)methyl)-4-methyl-1H-imidazol-5-yl)ethan-1-ol
38 N-((5-(1-aminoethyl)-4-methyl-1H-imidazol-2-yl)(3-chloro-4-fluorophenyl)methyl)-5-fluoro-6-methylpyridin-2-amine
39 N-((5-(2-amino-1,3-dimethoxypropan-2-yl)-1H-imidazol-2-yl)(3-chloro-4-fluorophenyl)methyl)-5-fluoro-6-methylpyridin-2-amine
40 N-((5-((azetidin-3-yloxy)methyl)-4-methyl-1H-imidazol-2-yl)(3-chloro-4-fluorophenyl)methyl)-5-fluoro-6-methylpyridin-2-amine
41 4-(2-((3-chloro-4-fluorophenyl)((4-(trifluoromethyl)cyclohexyl)oxy)methyl)-5-methyl-1H-imidazol-4-yl)piperidin-4-ol
42 4-(2-((3-chloro-4-fluorophenyl)((4-(trifluoromethyl)cyclohexyl)oxy)methyl)-5-(methoxymethyl)-1H-imidazol-4-yl)piperidin-4-ol
43 4-(2-((3-chloro-4-fluorophenyl)((4-(trifluoromethyl)cyclohexyl)oxy)methyl)-5-(methoxymethyl)-1H-imidazol-4-yl)-1-methylpiperidin-4-ol
44 4-(2-((3-chloro-4-fluorophenyl)((4-(trifluoromethyl)cyclohexyl)oxy)methyl)-5-methyl-1H-imidazol-4-yl)-1-methylpiperidin-4-ol
45 2-(2-(bis(3-chloro-4-fluorophenyl)methyl)-5-methyl-1H-imidazol-4-yl)-2-(dimethylamino)ethan-1-ol
46 2-amino-2-(2-((3-chloro-4-fluorophenyl)((5-fluoro-6-methylpyridin-2-yl)amino)methyl)-5-methyl-1H-imidazol-4-yl)ethan-1-ol
47 N-((4-(1-amino-2-methoxyethyl)-5-methyl-1H-imidazol-2-yl)(3-chloro-4-fluorophenyl)methyl)-5-fluoro-6-methylpyridin-2-amine
48 2-(2-((3-chloro-4-fluorophenyl)((5-fluoro-6-methylpyridin-2-yl)amino)methyl)-5-methyl-1H-imidazol-4-yl)-2-(methylamino)ethan-1-ol
49 2-(2-((3-chloro-4-fluorophenyl)((5-fluoro-6-methylpyridin-2-yl)amino)methyl)-5-methyl-1H-imidazol-4-yl)-2-(dimethylamino)ethan-1-ol
50 N-((3-chloro-4-fluorophenyl)(4-(1-(dimethylamino)-2-methoxyethyl)-5-methyl-1H-imidazol-2-yl)methyl)-5-fluoro-6-methylpyridin-2-amine
51 N-((4-(1-amino-2,2-difluoroethyl)-5-methyl-1H-imidazol-2-yl)(3-chloro-4-fluorophenyl)methyl)-5-fluoro-6-methylpyridin-2-amine
52 N-((4-(1-amino-2-(difluoromethoxy)ethyl)-5-methyl-1H-imidazol-2-yl)(3-chloro-4-fluorophenyl)methyl)-5-fluoro-6-methylpyridin-2-amine
53 N-((4-(1-amino-2-(trifluoromethoxy)ethyl)-5-methyl-1H-imidazol-2-yl)(3-chloro-4-fluorophenyl)methyl)-5-fluoro-6-methylpyridin-2-amine
54 2-amino-2-(2-((3-chloro-4-fluorophenyl)((4-(trifluoromethyl)cyclohexyl)oxy)methyl)-5-methyl-1H-imidazol-4-yl)ethan-1-ol
55 1-(2-((3-chloro-4-fluorophenyl)((4-(trifluoromethyl)cyclohexyl)oxy)methyl)-5-methyl-1H-imidazol-4-yl)-2-methoxyethan-1-amine
56 2-(2-((3-chloro-4-fluorophenyl)((4-(trifluoromethyl)cyclohexyl)oxy)methyl)-5-methyl-1H-imidazol-4-yl)-2-(dimethylamino)ethan-1-ol
57 1-(2-((3-chloro-4-fluorophenyl)((4-(trifluoromethyl)cyclohexyl)oxy)methyl)-5-methyl-1H-imidazol-4-yl)-2-(difluoromethoxy)ethan-1-amine
58 1-(2-((3-chloro-4-fluorophenyl)((4-(trifluoromethyl)cyclohexyl)oxy)methyl)-5-methyl-1H-imidazol-4-yl)-2-(trifluoromethoxy)ethan-1-amine
59 N-((3-chloro-4-fluorophenyl)(4-(2-methoxy-1-(methylamino)ethyl)-5-methyl-1H-imidazol-2-yl)methyl)-5-fluoro-6-methylpyridin-2-amine
60 2-amino-2-(2-(bis(4-chloro-2-methoxyphenyl)methyl)-5-methyl-1H-imidazol-4-yl)ethan-1-ol
61 1-(2-(bis(4-chloro-2-methoxyphenyl)methyl)-5-methyl-1H-imidazol-4-yl)-2-methoxyethan-1-amine
62 2-(2-(bis(4-chloro-2-methoxyphenyl)methyl)-5-methyl-1H-imidazol-4-yl)-2-(methylamino)ethan-1-ol
63 2-(2-(bis(4-chloro-2-methoxyphenyl)methyl)-5-methyl-1H-imidazol-4-yl)-2-(dimethylamino)ethan-1-ol
64 1-(2-(bis(4-chloro-2-methoxyphenyl)methyl)-5-methyl-1H-imidazol-4-yl)-2-(difluoromethoxy)ethan-1-amine
65 N-((3-chloro-4-fluorophenyl)(4-(2-(difluoromethoxy)-1-(methylamino)ethyl)-5-methyl-1H-imidazol-2-yl)methyl)-5-fluoro-6-methylpyridin-2-amine
66 2-amino-2-(2-(bis(3-chloro-4-fluorophenyl)methyl)-5-(difluoromethyl)-1H-imidazol-4-yl)ethan-1-ol
67 2-amino-2-(2-(bis(3-chloro-4-fluorophenyl)methyl)-5-(trifluoromethyl)-1H-imidazol-4-yl)ethan-1-ol
68 2-amino-2-(2-(bis(3-chloro-4-fluorophenyl)methyl)-5-chloro-1H-imidazol-4-yl)ethan-1-ol
69 2-amino-2-(2-((3-chloro-4-fluorophenyl)((5-fluoro-6-methylpyridin-2-yl)amino)methyl)-5-methyl-1H-imidazol-4-yl)propan-1-ol
70 4-(1-amino-2-hydroxyethyl)-2-(bis(3-chloro-4-fluorophenyl)methyl)-1H-imidazole-5-carbonitrile
and the physiologically acceptable salts thereof.

14. A medicament comprising a compound according to any of the preceding claims.
